# EUROPEAN PATENT SPECIFICATION

(11) **EP 2 211 643 B1**
(45) Date of publication and mention of the grant of the patent: **15.04.2015**
(21) Application number: 07859342.3
(22) Date of filing: 23.11.2007
(51) Int. Cl.: A23L 2/60, C12N 9/90, C12P 19/24

(54) **L-ARABINOSE ISOMERASE FOR CONVERTING D-GALACTOSE INTO D-TAGATOSE IN A DAIRY PRODUCT WHICH CONTAINS D-GALACTOSE**
L-ARABINOSE-ISOMERASE FÜR DIE UMWANDLUNG VON D-GALACTOSE IN D-TAGATOSE IN EINEM D-GALACTOSEHALTIGEN MILCHPRODUKT
L-ARABINOSE ISOMÉRASE POUR CONVERTIR DU D-GALACTOSE EN D-TAGATOSE DANS UN PRODUIT LAITIER QUI CONTIENT DU D-GALACTOSE

(43) Date of publication of application: 04.08.2010
(73) Proprietor: Institut National de la Recherche Agronomique (INRA), 75007 Paris (FR); Centre De Biotechnologie De Sfax, Sfax 3018 (TN)
(72) Inventor: RHIMI, Moez, 9110 Sidi Bouzid (TN); CHOUAYEKH, Hichem, 3021 Sfax (TN); MAGUIN, Emmanuelle, F-91440 Bures Sur Yvette (FR); BEJAR, Samir, 3058 Sfax (TN)
(74) Representative: Jacobson, Claude
(86) International application number: PCT/IB2007/004314
(87) International publication number: WO 2009/066127

(56) References cited:
- WO-A-03/008617
- WO-A2-2006/071203
- US-A- 6 057 135
- DATABASE KPOP [Online] Arabinose isomerase from Alicyclobacillus 21 February 2008 (2008-02-21), LEE S., LEE D., KIM B., CHOE E., HONG Y., KIM S., PYUN Y.R.: "Thermostable and acidophilic arabinose isomerase and process for preparing tagatose thereby." XP002499987 retrieved from EBI Database accession no. DI544843 -& KR 2004 0058544 A (PYUN YU RYANG) 5 July 2004 (2004-07-05)
- DATABASE UniProt [Online] L-arabinose isomerase EC Number:5.3.1.4 10 January 2006 (2006-01-10), LEE S.J., LEE D.W.,CHOE E.A., HONG Y.H., KIM S.B., PYUN Y.R.: "CHARACTERIZATION OF A THERMOACIDOPHILIC L-ARABINOSE ISOMERASE FROM A. ACIDOCALDARIUS:ROLE OF LYS-269 IN PH OPTIMUM" XP002499988 retrieved from EBI accession no. UNIPROT:Q2VMT2_ALIAC Database accession no. Q2VMT2 -& LEE SANG-JAE ET AL: "Characterization of a thermoacidophilic L-arabinose isomerase from Alicyclobacillus acidocaldarius: Role of Lys-269 in pH optimum" APPLIED AND ENVIRONMENTAL MICROBIOLOGY, vol. 71, no. 12, December 2005 (2005-12), pages 7888-7896, XP002499984 ISSN: 0099-2240
- KIM BYOUNG-CHAN ET AL: "Cloning, expression and characterization of L-arabinose isomerase from Thermotoga neapolitana: Bioconversion of D-galactose to D-tagatose using the enzyme" FEMS MICROBIOLOGY LETTERS, AMSTERDAM, NL, vol. 212, no. 1, 29 May 2002 (2002-05-29), pages 121-126, XP002387930 ISSN: 0378-1097
- HYO-JUNG OH ET AL: "Increase in D-Tagatose Production Rate by Site-directed Mutagenesis of L-Arabinose Isomerase from Geobacillus thermodenitrificans" BIOTECHNOLOGY LETTERS, KLUWER ACADEMIC PUBLISHERS, DO, vol. 28, no. 3, 1 February 2006 (2006-02-01), pages 145-149, XP019231191 ISSN: 1573-6776
- KIM JUNG-WOO ET AL: "Production of tagatose by a recombinant thermostable L-arabinose isomerase from Thermus sp. IM6501" BIOTECHNOLOGY LETTERS, KEW, SURREY, GB, vol. 25, no. 12, 1 June 2003 (2003-06-01), pages 963-967, XP002387925 ISSN: 0141-5492
- JORGENSEN F ET AL: "Enzymatic conversion of D-galactose to D-tagatose: heterologous expression and characterisation of a thermostable L-arabinose isomerase from Thermoanaerobacter mathranii" APPLIED MICROBIOLOGY AND BIOTECHNOLOGY, vol. 64, no. 6, June 2004 (2004-06), pages 816-822, XP002499985 ISSN: 0175-7598
- RYU SE-AH ET AL: "Continuous D-tagatose production by immobilized thermostable L-arabinose isomerase in a packed-bed bioreactor." BIOTECHNOLOGY PROGRESS, vol. 19, no. 6, November 2003 (2003-11), pages 1643-1647, XP002499986 ISSN: 8756-7938
- DEOK-KUN OH: "Tagatose: properties, applications, and biotechnological processes" APPLIED MICROBIOLOGY AND BIOTECHNOLOGY, SPRINGER, BERLIN, DE, vol. 76, no. 1, 10 May 2007 (2007-05-10), pages 1-8, XP019538798 ISSN: 1432-0614

### SEQUENCE LISTING

<110> INRA
<120> L-arabinose isomerase for converting D-galactose into D-tagatose in a dairy product which contains D-galactose
<130> BET 07P0458
<160> 10
<170> PatentIn version 3.4
<210> 1
   <211> 1491
   <212> DNA
   <213> Bacillus stearothermophilus strain US100
<220>
   <221> CDS
   <222> (1)..(1491)
   <223> L-arabinose isomerase
<400> 1
   atg ctg tca tta cgt cct tat gaa ttt tgg ttt gtg aca gga agt cag      48
   Met Leu Ser Leu Arg Pro Tyr Glu Phe Trp Phe Val Thr Gly Ser Gln
   1               5                   10                  15
   cac ttg tac gga gaa gaa gcg tta aaa caa gtc gaa gaa cat tca aga      96
   His Leu Tyr Gly Glu Glu Ala Leu Lys Gln Val Glu Glu His Ser Arg
               20                  25                  30
   atc atg gtc aat gaa tgg aat cgc gat tcg gtg ttt ccg ttc cca ttc     144
   Ile Met Val Asn Glu Trp Asn Arg Asp Ser Val Phe Pro Phe Pro Phe
           35                  40                  45
   gtt ttc aaa tca gtc gtg acg acg cca gag gaa atc cgg cgc gtt tgc     192
   Val Phe Lys Ser Val Val Thr Thr Pro Glu Glu Ile Arg Arg Val Cys
       50                  55                  60
   ctt gag gcg aat gcg agc gaa caa tgc gct ggg gtc atc act tgg atg     240
   Leu Glu Ala Asn Ala Ser Glu Gln Cys Ala Gly Val Ile Thr Trp Met
   65                  70                  75                  80
   cat aca ttc tcg cca gcg aaa atg tgg att ggc ggc ctt ttg gag ttg     288
   His Thr Phe Ser Pro Ala Lys Met Trp Ile Gly Gly Leu Leu Glu Leu
                   85                  90                  95
   aga aaa ccg tta ttg cat ctt cac acc cag ttt aac cgt gat att ccg     336
   Arg Lys Pro Leu Leu His Leu His Thr Gln Phe Asn Arg Asp Ile Pro
               100                 105                 110
   tgg gac agc atc gat atg gac ttt atg aac tta aac caa tcg gct cac     384
   Trp Asp Ser Ile Asp Met Asp Phe Met Asn Leu Asn Gln Ser Ala His
           115                 120                 125
   ggt gac cgg gaa tac gga ttt atc ggc gcg aga atg ggc gtg gcc cgg     432
   Gly Asp Arg Glu Tyr Gly Phe Ile Gly Ala Arg Met Gly Val Ala Arg
       130                 135                 140
   aaa gtg gtg gtc ggg cac tgg gaa gac cca gaa gtc cgc gag cgg ctg     480
   Lys Val Val Val Gly His Trp Glu Asp Pro Glu Val Arg Glu Arg Leu
   145                 150                 155                 160
   gcg aaa tgg atg cgg acg gct gtc gcg ttt gcg gaa agc cgc aac cta     528
   Ala Lys Trp Met Arg Thr Ala Val Ala Phe Ala Glu Ser Arg Asn Leu
                   165                 170                 175
   aaa gtg gct cgt ttt ggc gac aac atg cgt gaa gtg gct gtg acg gaa      576
   Lys Val Ala Arg Phe Gly Asp Asn Met Arg Glu Val Ala Val Thr Glu
               180                 185                 190
   ggg gac aaa gtc gga gcg caa att caa ttc ggc tgg tcg gtc aac ggc      624
   Gly Asp Lys Val Gly Ala Gln Ile Gln Phe Gly Trp Ser Val Asn Gly
           195                 200                 205
   tat ggc atc ggg gat ttg gtg caa tac atc cgc gat gtt tct gaa caa      672
   Tyr Gly Ile Gly Asp Leu Val Gln Tyr Ile Arg Asp Val Ser Glu Gln
       210                 215                 220
   aaa gtg aac gag ttg ctc gat gaa tac gag gag ctg tac gac att gta      720
   Lys Val Asn Glu Leu Leu Asp Glu Tyr Glu Glu Leu Tyr Asp Ile Val
   225                 230                 235                 240
   ccc gcc ggc cgc caa gaa ggg ccc gtt cgc gaa tca att cgt gaa cag      768
   Pro Ala Gly Arg Gln Glu Gly Pro Val Arg Glu Ser Ile Arg Glu Gln
                   245                 250                 255
   gcg cgg att gaa ctc ggg ctg aaa gcc ttt ttg caa gac ggg aac ttc      816
   Ala Arg Ile Glu Leu Gly Leu Lys Ala Phe Leu Gln Asp Gly Asn Phe
               260                 265                 270
   act gcc ttt acg acg acg ttt gaa gat ttg cac ggc atg aag caa ctt      864
   Thr Ala Phe Thr Thr Thr Phe Glu Asp Leu His Gly Met Lys Gln Leu
           275                 280                 285
   cca gga cta gcg gtt caa cgg ctt atg gca gag gga tat gga ttt ggc      912
   Pro Gly Leu Ala Val Gln Arg Leu Met Ala Glu Gly Tyr Gly Phe Gly
       290                 295                 300
   ggc gaa ggc gac tgg aaa acg gct gct ctc gtt cgg ttg atg aaa gtc      960
   Gly Glu Gly Asp Trp Lys Thr Ala Ala Leu Val Arg Leu Met Lys Val
   305                 310                 315                 320
   atg gcg gat ggc aaa gga aca tcg ttc atg gaa gac tac acg tac cac     1008
   Met Ala Asp Gly Lys Gly Thr Ser Phe Met Glu Asp Tyr Thr Tyr His
                   325                 330                 335
   ttt gag ctg ggc aac gaa ctg att ctt ggc gcg cat atg ctc gaa gta     1056
   Phe Glu Leu Gly Asn Glu Leu Ile Leu Gly Ala His Met Leu Glu Val
               340                 345                 350
   tgc ccg acg att gcc gca acc cgg ccg cgc atc gaa gtc cat ccg ctt     1104
   Cys Pro Thr Ile Ala Ala Thr Arg Pro Arg Ile Glu Val His Pro Leu
           355                 360                 365
   tcg atc ggt gga aaa gaa gat cca gcc cgt ctc gtg ttt gac ggc ggc     1152
   Ser Ile Gly Gly Lys Glu Asp Pro Ala Arg Leu Val Phe Asp Gly Gly
       370                 375                 380
   gag ggt gct gcg gtc aat gct tcg ttg att gac tta gga cac cgt ttc     1200
   Glu Gly Ala Ala Val Asn Ala Ser Leu Ile Asp Leu Gly His Arg Phe
   385                 390                 395                 400
   cgt ctc att gtc aat gaa gtc gat gca gtg aaa cca gaa cat gag atg     1248
   Arg Leu Ile Val Asn Glu Val Asp Ala Val Lys Pro Glu His Glu Met
                   405                 410                 415
   ccg aaa ctg cct gtt gcg cgc att cta tgg aaa ccg cgc ccg tca ctc     1296
   Pro Lys Leu Pro Val Ala Arg Ile Leu Trp Lys Pro Arg Pro Ser Leu
               420                 425                 430
   cgc gac tcg gcc gaa gca tgg att tta gcc ggc gga gcg cat cac aca     1344
   Arg Asp Ser Ala Glu Ala Trp Ile Leu Ala Gly Gly Ala His His Thr
           435                 440                 445
   tgc ttc tcg ttt gcg gtc acg gct gaa cag ctg caa gac ttt gcg gaa     1392
   Cys Phe Ser Phe Ala Val Thr Ala Glu Gln Leu Gln Asp Phe Ala Glu
       450                 455                 460
   atg gcg ggc att gaa tgc gtc gtg atc aat gaa cat acg tcc gtc tcc      1440
   Met Ala Gly Ile Glu Cys Val Val Ile Asn Glu His Thr Ser Val Ser
   465                 470                 475                 480
   tca ttc aag aac gaa cta aga tgg aat gaa gta ttc tgg cgg ggg cgg      1488
   Ser Phe Lys Asn Glu Leu Arg Trp Asn Glu Val Phe Trp Arg Gly Arg
                   485                 490                 495
   taa                                                                  1491
<210> 2
   <211> 496
   <212> PRT
   <213> Bacillus stearothermophilus strain US100
<400> 2
   Met Leu Ser Leu Arg Pro Tyr Glu Phe Trp Phe Val Thr Gly Ser Gln
   1               5                   10                  15
   His Leu Tyr Gly Glu Glu Ala Leu Lys Gln Val Glu Glu His Ser Arg
               20                  25                  30
   Ile Met Val Asn Glu Trp Asn Arg Asp Ser Val Phe Pro Phe Pro Phe
           35                  40                  45
   Val Phe Lys Ser Val Val Thr Thr Pro Glu Glu Ile Arg Arg Val Cys
       50                  55                  60
   Leu Glu Ala Asn Ala Ser Glu Gln Cys Ala Gly Val Ile Thr Trp Met
   65                  70                  75                  80
   His Thr Phe Ser Pro Ala Lys Met Trp Ile Gly Gly Leu Leu Glu Leu
                   85                  90                  95
   Arg Lys Pro Leu Leu His Leu His Thr Gln Phe Asn Arg Asp Ile Pro
               100                 105                 110
   Trp Asp Ser Ile Asp Met Asp Phe Met Asn Leu Asn Gln Ser Ala His
           115                 120                 125
   Gly Asp Arg Glu Tyr Gly Phe Ile Gly Ala Arg Met Gly Val Ala Arg
       130                 135                 140
   Lys Val Val Val Gly His Trp Glu Asp Pro Glu Val Arg Glu Arg Leu
   145                 150                 155                 160
   Ala Lys Trp Met Arg Thr Ala Val Ala Phe Ala Glu Ser Arg Asn Leu
                   165                 170                 175
   Lys Val Ala Arg Phe Gly Asp Asn Met Arg Glu Val Ala Val Thr Glu
               180                 185                 190
   Gly Asp Lys Val Gly Ala Gln Ile Gln Phe Gly Trp Ser Val Asn Gly
   195 200 205
   Tyr Gly Ile Gly Asp Leu Val Gln Tyr Ile Arg Asp Val Ser Glu Gln
   210 215 220
   Lys Val Asn Glu Leu Leu Asp Glu Tyr Glu Glu Leu Tyr Asp Ile Val
   225 230 235 240
   Pro Ala Gly Arg Gln Glu Gly Pro Val Arg Glu Ser Ile Arg Glu Gln
   245 250 255
   Ala Arg Ile Glu Leu Gly Leu Lys Ala Phe Leu Gln Asp Gly Asn Phe
   260 265 270
   Thr Ala Phe Thr Thr Thr Phe Glu Asp Leu His Gly Met Lys Gln Leu
   275 280 285
   Pro Gly Leu Ala Val Gln Arg Leu Met Ala Glu Gly Tyr Gly Phe Gly
   290 295 300
   Gly Glu Gly Asp Trp Lys Thr Ala Ala Leu Val Arg Leu Met Lys Val
   305 310 315 320
   Met Ala Asp Gly Lys Gly Thr Ser Phe Met Glu Asp Tyr Thr Tyr His
   325 330 335
   Phe Glu Leu Gly Asn Glu Leu Ile Leu Gly Ala His Met Leu Glu Val
   340 345 350
   Cys Pro Thr Ile Ala Ala Thr Arg Pro Arg Ile Glu Val His Pro Leu
   355 360 365
   Ser Ile Gly Gly Lys Glu Asp Pro Ala Arg Leu Val Phe Asp Gly Gly
   370 375 380
   Glu Gly Ala Ala Val Asn Ala Ser Leu Ile Asp Leu Gly His Arg Phe
   385 390 395 400
   Arg Leu Ile Val Asn Glu Val Asp Ala Val Lys Pro Glu His Glu Met
   405 410 415
   Pro Lys Leu Pro Val Ala Arg Ile Leu Trp Lys Pro Arg Pro Ser Leu
   420 425 430
   Arg Asp Ser Ala Glu Ala Trp Ile Leu Ala Gly Gly Ala His His Thr
   435 440 445
   Cys Phe Ser Phe Ala Val Thr Ala Glu Gln Leu Gln Asp Phe Ala Glu
   450 455 460
   Met Ala Gly Ile Glu Cys Val Val Ile Asn Glu His Thr Ser Val Ser
   465 470 475 480
   Ser Phe Lys Asn Glu Leu Arg Trp Asn Glu Val Phe Trp Arg Gly Arg
   485 490 495
<210> 3
   <211> 20
   <212> DNA
   <213> Artificial
<220>
   <223> primer
<400> 3
   gtgaacgggg aggagcaatg 20
<210> 4
   <211> 20
   <212> DNA
   <213> Artificial
<220>
   <223> primer
<400> 4
   gaaatcttac cgcccccgcc 20
<210> 5
   <211> 21
   <212> DNA
   <213> Artificial
<220>
   <223> primer
<400> 5
   gcctttttga aagacgggaa c 21
<210> 6
   <211> 21
   <212> DNA
   <213> Artificial
<220>
   <223> primer
<400> 6
   gttcccgtct ttcaaaaagg c 21
<210> 7
   <211> 32
   <212> DNA
   <213> Artificial
<220>
   <223> primer
<400> 7
   gaatggaatc gcgatccggt gttccgttcc ca 32
<210> 8
   <211> 32
   <212> DNA
   <213> Artificial
<220>
   <223> primer
<400> 8
   tgggaacgaa acaccggatc gcgattccat tc 32
<210> 9
   <211> 36
   <212> DNA
   <213> Artificial
<220>
   <223> primer
<400> 9
   gcgtttgcgg aaagccgcaa cctaaaagtg gctcgt 36
<210> 10
   <211> 36
   <212> DNA
   <213> Artificial
<220>
   <223> primer
<400> 10
   acgagccact tttaggttgc ggctttccgc aaacgc 36

## Description

The instant invention relates to the use of a L-arabinose isomerase for converting D-galactose into D-tagatose in a dairy product which contains D-galactose, wherein said dairy product containing D-galactose is selected from the group consisting of milk, milk supplemented with D-galactose, mild undergoing lactic acid fermentation, a fermented mild, and milk in which lactose was enzymatically processed, to a method of converting D-galactose into D-tagatose during lactic acid fermentation of milk, to a method of producing a sweetened fermented dairy product, and to a sweetening milk product additive which comprises a L-arabinose isomerase and/or a L-arabinose isomerase-expressing microorganism acceptable for food consumption. The invention also relates to a microorganism acceptable for food consumption expressing an exogenous L-arabinose isomerase, and to a mutated L-arabinose isomerase US100 from *Bacillus stearothermophilus,* in particular to a mutant with enhanced acidotolerance and stability at acid pH or to a mutant with lower optimal temperature.

Lactic acid bacteria (LAB) constitute a commercially important group of microorganisms extensively utilized in the food industry. Among them, the dairy starters *Lactobacillus delbrueckii* subsp. *bulgaricus* (*L. bulgaricus)* and *Streptococcus thermophilus* are universally used in cooperation in the fermentation of milk into yoghurt (Tamine and Robinson, 1999, Yoghurt Science and Technology, 2nd edn. Cambridge: Woodhead). During their growth, lactose the unique sugar found in milk is used as the primary energy source.

In LAB, two systems for transport and metabolism of lactose are known: (i) a phosphoenolpyruvate (PEP) lactose phosphotransferase system (PTS) with a phospho-β-galactosidase system (Postma et al., 1993, Microbiol Rev 57:543-94) and (ii) a lactose antiport permease (LacS) system with a β-galactosidase (LacZ). The PEP-PTS system is found in many species including lactococci (Reizer et al., 1988. Crit Rev Microbiol, 15:297-338; Thompson, 1987, FEMS Microb. Lett, 46:221-23). *L. bulgaricus* as well as S. *thermophilus* utilize lactose via the second pathway where the lactose is brought into cell as the free sugar and cleaved by β-galactosidase into glucose and galactose (Foucaud and Poolman, 1992, J Biol Chem, 267:22087-94; Leong-Morgenthaler et al., 1991, J Bacteriol, 173:1951-7). The glucose moiety of the generated monosaccharide is further metabolized to pyruvate which is used to produce lactate, whereas the galactose moiety is excreted from most strains of these species into the medium in equimolar amounts with the lactose uptake despite the fact that these starters contain genes of the Leloir pathway which is the most ubiquitous route for galactose catabolism in these bacteria (de Vin et al., 2005. Appl Environ Microbiol 71:3659-67; Hickey, et al., 1986. Appl Environ Microbiol 51:825-831; Hutkins and Ponne, 1991. Appl Environ Microbiol 57:941-944). Currently, the galactose negative phenotype has been mainly ascribed to a defect in the induction mechanism of enzymes of the Leloir pathway during growth on lactose and to the preferred mode of action antiport of the permease LacS *in vivo* (de Vin et al., 2005. Appl Environ Microbiol 71:3659-67; Turner and Martley. 1983. Appl Environ Microbiol 45:1932-1934).

Recently milk with lower lactose concentration was also proposed for lactose-intolerant persons. Lactose hydrolysis can achieve this decrease in lactose concentration leading to glucose and galactose. The Inventors showed that L-AI can be used to convert the galactose resulting from lactose hydrolysis in the low-calorie, sweetening D-tagatose.

In yoghurt manufacture, especially in the case of fruit/flavored products, sweetening compounds with high caloric value like sucrose are normally added to tone down the acidity of the product. The fact that nowadays we assist to an increasing of consumer demand for low caloric food products especially in the case of diabetes and low caloric diet persons, the actual chalenge is to try to convert the unconsumed D-galactose during yoghurt production to low caloric sweetener like D-tagatose which could minimize the habitual adding of high caloric sugars. This natural D-galactose isomer, is a rare ketohexose with a sweetening power equivalent to sucrose but a much lower caloric value because it is poorly degraded by the human body, making it an interesting anti-hyperglycemiant agent.

There is no biological source available for the rentable extraction of tagatose. Two processes have been described for the biological production of this sweetener. The first one relies on the ability of many micro-organisms, such as *Arthrobacter, Lactobacillus, Mycobacterium, Klebsiella* and *Gluconobacter,* to convert D-galactitol into D-tagatose (Muniruzzaman et al., J. ferment. Bioeng. 78 (1994) 145-148.; Shimonishi et al., J. Ferment. Bioeng. 79 (1995) 620-622.; Manzoni et al., Pro. Biochem. 36 (2001) 917-977; Rollini and Manzoni, Proc. Biochem. 40 (2005) 437-444). However, it is not of industrial use because the D-galactitol is an expensive substrate which has a low potential for industrial application. The second one is an in vitro enzymatic isomerisation of D-galactose using an L-arabinose isomerase (L-AI) activity (Haltrich et al., 1998. Ann N Y Acad Sci 864:295-9; Izumori et al., 1978. J Bacteriol 133:413-4; Jorgensen et al., 2004. Appl Microbiol Biotechnol 64:816-22; Manzoni et al., 2001. Process biochemistry 36:971-977). This enzyme is involved, *in vivo,* in the isomerisation of L-arabinose to L-ribulose.

The L-arabinose isomerase derived from *B. stearothermophilus* US100 (L-AI US100) has been characterized (international patent application WO 2006/071203; Rhimi and Bejar. 2006. Biochim Biophys Acta 1760:191-9). L-AI US100 is optimally active at pH 7.5 and 80°C and, by contrast with the L-AI previously characterized, exhibits a low requirement for metal ions.

The inventors have demonstrated that L-AI US100 produced by lactic acid bacteria is active *in vivo* since it conferred an efficient bioconversion of D-galactose to D-tagatose which is secreted from the microbial cells into culture medium. In addition, purified L-AI US100 enzyme was also found to convert efficiently D-galactose into D-tagatose in milk, the industrial medium used for yoghurt production and also several probiotic products. These results provides for the exploitation of L-AI activity to produce new low caloric milk products containing D-tagatose produced by enzymatic isomerisation.

Isomerization of D-galactose by L-arabinose isomerases at high temperature increases the reaction rate and allows the shift of equilibrium between D-galactose and D-tagatose toward the latter. For this reason, many thermostable L-Als were isolated and characterized including those from *Thermus, Thermotoga* and *Geobacillus* genera. Nevertheless, the majority of these enzymes have a high optimum pH which is a major drawback with respect to an application during lactic acid fermentation, wherein milk pH will be remote from the optimum pH of the enzyme.

Protein engineering of new acidotolerant L-arabinose isomerase was previously explored in order to found L-Als active at acid pH. In fact, Kim et al 2006 reported that V408R and N475Q substitutions allow the shift of the *Geobacillus stearothermophilus* T6 L-AI optimal pH from 8.5 to 7.5 (J Appl Microbiol 101 (2006) 213). Moreover, Lee et al. described for the first time the characterisation of a new acidotolerant L-AI from the acidophilic *Alicyclobacillus acidocaldarius* (Appl Environ Microbiol 71 (2005) 7888). However, while this mutant L-AI is acidolerant, it requires divalent ions both for its activity and thermal stability (1 mM Co²⁺, 1 mM Mn²⁺ and 1 mM Mg²⁺) and has a relative lower optimal temperature evaluated to 65°C. Moreover, nostructural explanation was proposed to explain the impact of these substitutions on the enzyme acidotolerance behaviour.

New L-AI acting at higher or lower temperature, low pH and displaying a very feeble requirement for metallic ions for their activity and thermal stability were looked for. A new acidotolerant L-AI US100 Q268K mutant, a mesophile L-AI US100 N175H mutant and an acidotolerant and mesophile double mutant L-AI US100 Q268K N175H were constructed, purified, and characterised, in particular as regards D-tagatose production.

The invention is defined by the claims.

### L-arabinose isomerases

L-arabinose isomerases (EC 5.3.1.4) are intracellular enzymes that catalyse the conversion of L-arabinose to L-ribulose in biological systems. They are also referred to as D-galactose isomerases due to their ability, *in vitro,* to isomerize D-galactose into D-tagatose (Cheetam and Woottom, Enzyme and Microbial Technology 15 (1993) 105).

In the context of the invention, the term "L-arabinose isomerase" or "L-AI" generally denotes any enzyme which catalyses the conversion of L-arabinose to L-ribulose, and preferably also isomerizes D-galactose into D-tagatose. A L-arabinose isomerase may be a native (or wild-type) L-AI, or a mutant of a native L-AI which was genetically engineered to alter one or more phenotypic features of the native L-AI, in particular temperature optimum, pH optimum, ion requirement, substrate affinity, thermal stability and/or pH tolerance, provided said mutant retains the capacity to isomerize D-galactose into D-tagatose, and optionally to convert L arabinose into L ribulose.

The capacity of an enzyme to generate L-ribulose or D-tagatose may be readily determined by the skilled in the art with the cysteine carbazole sulfuric-acid method which was described by Dische and Borenfreund (J. Biol. Chem. 192 (1951) 583), with absorbance measured at 560 nm. The L-ribulose or D-tagatose production may also be detected by high-performance ionic chromatography (HPLC).

L-arabinose isomerases are expressed by a wide number of organisms, including plants such as Japanese rice, and microorganisms such as yeasts or bacteria.

In particular, numerous bacterial L-arabinose isomerases (L-Als) have been isolated and described, and some have been deposited in sequence databases. For instance, L-arabinose isomerases (L-Als) may derive from :
- a bacterium of the genus *Thermus* (Thermoanaerobacter mathranii), of the genus *Thermotoga* (*Thermotoga maritima* (UniProtKB/Swiss-Prot entry Q9WYB3), *Thermotoga neapolitana*),
- a bacterium of the genus *Geobacillus (Geobacillus stearothermophilus, Geobacillus thermodenitrificans, Geobacillus kaustophilus* UniProtKB/Swiss-Prot entry Q5KYP7),
- *Alicyclobacillus acidocaldarius,*
- a bacterium of the genus *Bacillus (Bacillus stearothermophilus* (UniProtKB/Swiss-Prot entry Q9S467), *Bacillus licheniformis* (UniProtKB/Swiss-Prot entry Q65GC0 Q65J10), *Bacillus clausii* (UniProtKB/Swiss-Prot entry Q5WL05), *Bacillus halodurans* (UniProtKB/Swiss-Prot entry Q9KBQ2), *Bacillus subtilis* (UniProtKB/Swiss-Prot entry P94523),
- *Clostridium acetobutylicum,*
- *E. coli* (UniProtKB/Swiss-Prot entry P58538, P08202, Q0TLS8, Q1 RGD7, Q8FL89),
- *Mycobacterium smegmatis* (UniProtKB/Swiss-Prot entry Q9RHG2),
- a bacterium of the genus *Salmonella (Salmonella paratyphi-a* (UniProtKB/Swiss-Prot entry Q5PDF2), *Salmonella Typhi* (UniProtKB/Swiss-Prot entry P58539), *Salmonella Choleraesuis* (UniProtKB/Swiss-Prot entry Q57TF9), *Salmonella typhimurium* (UniProtKB/Swiss-Prot entry P06189), *Shigella bodyii* (UniProtKB/Swiss-Prot entry Q326H3),
- a bacterium of the genus *Shigella (Shigella soneii* (UniProtKB/Swiss-Prot entry Q3Z5U9), *Shigella dysenteriae* (UniProtKB/Swiss-Prot entry Q32K31), *Shigella flexneri* (UniProtKB/Swiss-Prot entry Q7UDT4),
- a bacterium of the genus *Yersinia (Yersinia pestis* (UniProtKB/Swiss-Prot entry P58540, Q1CIX9, Q1C7J3), *Yersinia pseudotuberculosis* (UniProtKB/Swiss-Prot entry Q66AF8),
- *Acidobacteria bacterium* (UniProtKB/Swiss-Prot entry Q1IUW9),
- *Klebsiella pneumoniae* (UniProtKB/Swiss-Prot entry Q48433),
- *Oceanobacillus iheyensis* (UniProtKB/Swiss-Prot entry Q8EMP4),
- *Erwinia carotovora* (UniProtKB/Swiss-Prot entry Q6D4W5), or
- *Mannheimia succiniciproducens* (UniProtKB/Swiss-Prot entry Q65WJ5).

A L-arabinose isomerase (L-AI) according to the invention may derive from any organism or microorganism, in particular any of the above bacteria, and more generally from any human or animal commensal micro-organism. For instance, said L-AI may derive from a thermophilic bacterium, in particular a bacterium of the genus *Thermus (Thermoanaerobacter mathranii*), of the genus *Thermotoga (Thermotoga maritima, Thermotoga neapolitana),* of the genus *Geobacillus (Geobacillus stearothermophilus, Geobacillus thermodenitrificans),* from *Alicyclobacillus acidocaldarius,* and from the genus *Bacillus (Bacillus stearothermophilus).* Said L-AI may also derive from a lactic acid bacterium, i.e. a bacterium belonging to the genera *Aerococcus, Alloicoccus, Atopobium, Bifidobacterium, Carnobacterium, Enterococcus, Lactobacillus, Lactococcus, Lactosphaera, Leuconostoc, Melissococcus, Microbacterium, Oenococcus Pediococcus, Propionibacterium, Streptococcus, Tetragenococcus, Vagococcus,* and *Weissella.*

As used herein, a L-arabinose isomerase "deriving" from e.g a bacterium, is meant for a L-AI isolated from a bacterium (native or wild-type L-AI), or mutants thereof that retain the capacity to convert D-galactose into D-tagatose and optionally to isomerize L-arabinose into L-ribulose.

The capacity to convert D-galactose into D-tagatose and optionally to isomerize L-arabinose into L-ribulose may be detected as described in the following examples. Briefly, a conversion or isomerization assay may be performed at the optimal temperature for the enzyme, e.g. 80°C, for 1 min for L-arabinose or 10 min for D-galactose, in a reaction mixture containing 0.2 mM CoCl₂, 1 mM MnSO₄, 50 □µl of crude enzyme extract at a suitable dilution, 5 mM of L-arabinose (or D-galactose) and MOPS buffer 100 mM (pH 7.5) up to a final volume of 1 ml. The reaction mixture is incubated. Then, conversion/isomerase activity may be measured by determining the amount of produced L-ribulose or D-tagatose by the cysteine carbazole sulfuric-acid method (Dische and Borenfreund. 1951. J Biol Chem 192:583-7), and measurement of absorbance at 560 nm. However, any other suitable method known by the skilled in the art, such as chromatography, may be used.

The L-arabinose isomerase may derive from *Alicyclobacillus acidocaldarius.* More specifically, said L-arabinose isomerase may be the *Alicyclobacillus acidocaldarius* L-AI cloned by Lee et al. (2005, Appl. Environ. Microbiol. 71 (12), 7888-7896; "AAAI", GenPept accession number AAY68209), or its K269E mutant (AAAI-K269E).

The L-arabinose isomerase may also derive from *Bacillus halodurans.* More specifically, said L-arabinose isomerase may be the *Bacillus halodurans* L-AI described in Lee et al. (2005, Appl. Environ. Microbiol. 71 (12), 7888-7896; "BHAI" UniProtKB/Swiss-Prot entry Q9KBQ2), or its E268K mutant (BHAI-E268K).

The L-arabinose isomerase may also derive from the *Bacillus stearothermophilus* strain US100. Said L-arabinose isomerase may be L-AI US100 as was described by Rhimi and Bejar (Biochim Biophys Acta. 2006 Feb;1760(2):191-9; international patent application WO 2006/071203). The polynucleotide sequence of L-AI US100 is shown in SEQ ID NO:1 (GenBank accession number AJ866972), and its amino acid sequence is shown in SEQ ID NO:2.

Said L-arabinose isomerase may also be a Q268K mutant of L-AI US100, i.e. a mutated L-AI US100 comprising a substitution Q to K at position 268 of SEQ ID NO:2. Indeed, as described herein, a mutant Q268K of L-AI US100 has been engineered. The biochemical and kinetic characterizations showed that this mutant is more acidotolerant and more stable at acid pH than wild-type L-AI US100, and that this mutant does not require addition of metal ions to operate production of D-tagatose.

Furthermore, a mutant N175H of L-AI US100 was developed which was found to show improved mesophily as compared with wild-type L-AI US100. The double mutant L-AI US100 Q268K N175H was further found to cumulate both improved acidotolerance and improved mesophily.

Thus, the invention relates also to a mutated L-AI US100 comprising (i) a substitution Q to K at position 268 of SEQ ID NO:2, or (ii) a substitution N to H at position 175 of SEQ ID NO:2, or (iii) a substitution N to H at position 175 of SEQ ID NO:2 and a substitution Q to K at position 268 of SEQ ID NO:2 and their coding sequences.

Accordingly the invention provides an isolated polypeptide comprising, or consisting of, the amino acid sequence shown in SEO ID NO:2 wherein (i) Q268 is substituted by K (Q268K), or (ii) N175 is substituted by H (N175H), or (iii) Q268 is substituted by K and N175 is substituted by H (Q268K N175H).

The invention also relates to an isolated nucleic acid comprising, or consisting of, a sequence encoding the amino acid sequence shown in SEQ ID NO:2 wherein (i) Q268 is substituted by K, or (ii) N175 is substituted by H, or (iii) Q268 is substituted by K and N175 is substituted by H. In particular, said isolated nucleic acid may comprise, or consists of, the sequence SEQ ID NO:1 wherein (i) C at position 802 is substituted by A (802C>A), or (ii) A at position 523 is substituted by C (523A>C), or (iii) C at position 802 is substituted by A and A at position 523 is substituted by C (802C>A, 523A>C).

Pursuant to the nomenclature of Dunnen and Antonarakis (Hum Mutation. 2000; 15 :7-12), amino acid substitutions are designated for instant by "Q268K", which means that Gln in position 268 is replaced by Lys. At the nucleic acid level, substitutions are designated by ">", e.g. "802C>A" denotes that at nucleotide 802 of the reference sequence a C is changed to a A.

### Microorganisms expressing L-arabinose isomerase

The application also discloses a microorganism which expresses a L-arabinose isomerase (L-AI). The microorganism is preferably a bacterium or a yeast.

Preferably, said microorganism is acceptable for food consumption. As used herein, a microorganism, in particular a bacterium or yeast, "acceptable for food consumption" denotes a microorganism which may be added into a milk intended for the manufacture of dairy products (in particular fermented dairy products) for human consumption.

The invention relates to a bacterium acceptable for food consumption which expresses an exogenous L-arabinose isomerase.

A bacterium acceptable for food consumption may be a lactic acid bacterium (LAB).

As used herein, "lactic acid bacteria" denote lactic acid-producing Gram-positive bacteria belonging to the genera *Aerococcus, Alloicoccus, Atopobium, Bifidobacterium, Carnobacterium, Enterococcus, Lactobacillus, Lactococcus, Lactosphaera, Leuconostoc, Melissococcus, Microbacterium, Oenococcus Pediococcus, Propionibacterium, Streptococcus, Tetragenococcus, Vagococcus,* and *Weissella.* In the context of the invention, the LAB will preferably be a bacterium Generally Recognised As Safe (GRAS).

Industrially, the most useful lactic acid bacteria are species of the genus *Lactococcus, Streptococcus, Lactobacillus, Leuconostoc, Bifidobacterium* and *Pediococcus.* Preferably, said lactic acid bacterium is selected from the group consisting of *Bifidobacterium bifidus, Bifidobacterium longum; Leuconostoc mesenteroides, Pediococcus cerevisiae, Lactococcus lactis, Lactobacillus bulgaricus, Lactobacillus sakei, Lactobacillus fermentum* and *Streptococcus thermophilus.* Some lactic acid bacteria are considered as "probiotics" (i.e. as providing benefit to health): for instance *Lactobacillus acidophilus, Lactobacillus casei, Lactobacillus plantarum, Lactobacillus rhamnosus, Lactobacillus johnsonii, Lactobacillus reuteri, Lactobacillus gasseri, Bifidobacterium longum, Bifidobacterium breve, Bifidobacterium bifidum,* and *Bifidobacterium infantis.*

The microorganism, in particular the microorganism acceptable for food consumption, may also be a non-lactic acid bacterium, for instance a non-lactic acid probiotic bacterium, or more generally a probiotic microorganism, such as a yeast.

The L-AI-expressing microorganism, especially the L-AI-expressing bacterium, may naturally (i.e. intrinsically) express the L-AI. In such as case said L-AI is said to be endogenous to the microorganism. This is the case for instance of *Lactobacillus sakei,* and *Lactobacillus plantarum.*

The L-arabinose isomerase may also be exogenous, i.e. foreign or extrinsic, to the microorganism. A microorganism, such as a bacterium or a yeast, expressing an exogenous L-arabinose isomerase may be readily prepared by the skilled in the art by conventional genetic engineering methods. In particular, the bacterium or yeast may be transformed with a vector suitable for transformation of a bacterium or yeast, as appropriate, which vector comprises a nucleic acid sequence encoding a L-arabinose isomerase. Where the microorganism is a bacterium, any other suitable method may be used to introduce a sequence encoding a L-arabinose isomerase in the bacterium, for instance bacterial conjugation, of phage transfer.

As used herein, the term "transformation" generally means the introduction of a foreign gene, DNA or RNA sequence into a host cell, so that the host cell will express the introduced gene or sequence to produce a desired substance, typically a protein or enzyme coded by the introduced gene or sequence. As used herein, a host cell which has been "transformed" with a nucleic acid sequence encoding a L-arabinose isomerase denotes a cell that receives and expresses an introduced DNA or RNA encoding a L-AI, whatever the method used to introduce said DNA or RNA (e.g. transformation with a vector, bacterial conjugation, via phage transfer, transposition, homologous recombination).

The terms "vector", "cloning vector" and "expression vector" mean the vehicle by which a DNA or RNA sequence (e.g. a foreign gene) can be introduced into a host cell, so as to transform the host and promote expression of the introduced sequence. A common type of vector is a "plasmid", which generally is a self-contained molecule of doublestranded DNA, usually of bacterial origin, that can readily accept additional (foreign) DNA and which can readily be introduced into a suitable host cell. A plasmid vector often contains coding DNA and promoter DNA and has one or more restriction sites suitable for inserting foreign DNA. A large number of vectors have been described for replication and/or expression in a variety of prokaryotic hosts. Non-limiting examples include pKK plasmids (Clonetech), pUC plasmids, pET plasmids (Novagen, Inc., Madison, WI), pRSET or pREP plasmids (Invitrogen, San Diego, CA), or pMAL plasmids (New England Biolabs, Beverly, MA).

Accordingly, the application further discloses a vector suitable for transformation of a microorganism, in particular a bacterium or yeast acceptable for food consumption, which comprises a nucleic acid sequence encoding a L-arabinose isomerase.

In said vector, the nucleic acid sequence encoding a L-arabinose isomerase may be placed under the control of a strong and constitutive promoter, for instance the promoter of *L. bulgaricus* ATCC 11842 *hlbA* gene. Inducible promoters may also be used.

The sequence encoding a L-arabinose isomerase may also be fused with a secretion signal directing secretion of the encoded polypeptide.

The encoded L-arabinose isomerase may be any L-AI, as described above. In particular, the L-AI may derive from a bacterium selected from the group consisting of a bacterium of the genus *Thermus (Thermoanaerobacter mathranii*), of the genus *Thermotoga (Thermotoga maritima, Thermotoga neapolitana),* of the genus *Geobacillus (Geobacillus stearothermophilus, Geobacillus thermodenitrificans),* from *Alicyclobacillus acidocaldarius,* from of the genus *Lactobacillus* (in particular *Lactobacillus sakei, Lactobacillus plantarum),* from the genus *Bacillus (Bacillus stearothermophilus) and from* a lactic acid bacterium.

The vector according to the invention comprises a nucleic acid sequence encoding an L-arabinose isomerase selected from the group consisting of AAAI, AAAI-K269E, BHAI, BHAI-E268K, L-AI US 100, and L-AI US 100 mutants L-AI US100-Q268K, L-AI US100-N175H and L-AI US100- (Q268K, N175H).

The invention also encompasses the bacterium acceptable for food consumption, transformed with (i.e. which comprises) one of the vectors as described above, or which comprises a nucleic acid sequence encoding a L-arabinose isomerase as defined above.

### Conversion of D-galactose into D-tagatose in milk

Conceptually, conversion of D-galactose into D-tagatose by L-arabinose isomerase directly in the milk, in particular during lactic acid fermentation, could have been hampered by several parameters:
- milk is a fat, complex medium, and it was unpredictable whether milk components could inactivate L-arabinose isomerase, where the enzyme is directly added into the milk;
- lactic acid bacteria, in particular *L. bulgaricus* and S. *thermophilus,* are galactose negative bacteria that excrete galactose into their culture medium during lactic acid fermentation of milk. Since L-arabinose isomerases are intracellular enzymes, it was unknown whether bioconversion of extracellular D-galactose into D-tagatose by a L-AI expressing bacterium would be feasible, due to a possible lack of accessibility of the enzyme to its substrate;
- during lactic acid fermentation, pH of milk switches from 6.5-6.8 to about 4.5 to5.5. As the optimal pH of L-Als, and in particular of the model enzyme used by the inventors (L-AI of *Bacillus stearothermophilus* US 100), is remote from the pH reached in the course of lactic acid fermentation, it was questionable whether the rate of D-galactose conversion into D-tagatose could be significant.

However, the inventors demonstrated that L-AI can successfully ensure conversion of D-galactose into D-tagatose in milk, especially during lactic acid fermentation of milk.

Accordingly, the invention relates to the use of a L-arabinose isomerase (L-AI) for converting D-galactose into D-tagatose in a dairy product which contains D-galactose, wherein said dairy product containing D-galactose is selected from the group consisting of milk, milk supplemented with D-galactose, mild undergoing lactic acid fermentation, a fermented mild, and milk in which lactose was enzymatically processed.

A dairy product generally denotes a foodstuff produced from milk and includes milk (e.g. milk optionally partly or totaly skimmed and/or homogenized and/or pasteurized). A dairy product which contains D-galactose may include milk supplemented with D-galactose, milk undergoing lactic acid fermentation, fermented milk, or milk in which lactose was processed into glucose and galactose (e.g. milk treated with a lactase, such as a β-galactosidase in order to hydrolyse enzymatically lactose).

In particular, the invention may relate to the use of a L-arabinose isomerase (L-AI) for converting D-galactose into D-tagatose during lactic acid fermentation of milk or in a fermented milk.

The L-arabinose isomerase may be any L-arabinose isomerase, as described above.

Conversion of D-galactose into D-tagatose may be conducted by adding a L-AI, or L-AI-expressing microorganism, directly into the dairy product containing D-galactose.

Conversion may also be conducted by contacting the dairy product containing D-galactose with a L-AI or L-AI-expressing microorganism. According to this embodiment, the L-AI or microorganism may be recovered from the end product, hence is absent from the end product. Contacting may be performed, for instance, by adding into the dairy product a L-AI or L-AI expressing microorganism trapped in a solid structure, for instance in alginate beads, and by removing the solid structure, preferably when D-galactose conversion is sufficient to obtain the desired property. Contacting may also be performed by contacting the dairy product with a L-AI or a L-AI-expressing microorganism, immobilised on a solid support such as a column or a reactor.

According to an embodiment, at least one L-arabinose isomerase is added into the dairy product. Where the dairy product is milk to be fermented, addition of the L-arabinose isomerase into the milk may be performed before beginning of lactic acid fermentation, in the course or at the end of lactic acid fermentation.

The skilled person may readily determine the quantity of L-arabinose isomerase which may be added into the dairy product, in particular in a milk intended for lactic acid fermentation. For instance, the quantity of L-AI added may be comprised between 0.1-20 mg/ml (mg L-AI/ml milk), preferably 0.5-10 mg/ml, more preferably 1-5 mg/ml, still preferably 2-3 mg/ml.

According to another embodiment, at least one L-arabinose isomerase-expressing microorganism is added into the dairy product. Said microorganism is preferably a bacterium, in particular a bacterium acceptable for food consumption. Where the dairy product is milk to be fermented, addition of the L-AI expressing microorganism into the milk may be performed before beginning of lactic acid fermentation, in the course or at the end of lactic acid fermentation.

L-arabinose isomerase-expressing microorganisms have been defined above. It may be a microorganism which was transformed to express L-arabinose isomerase. Alternatively, the L-arabinose isomerase-expressing microorganism may naturally express L-arabinose isomerase.

The quantity of microorganism which may be added in the dairy product may be readily determined by the skilled person. In particular, the level of L-AI activity of the microorganism in the dairy product may be assessed by using the cysteine carbazole sulfuric-acid method according to Dische and Borenfreund (J. Biol. Chem. 192 (1951) 583), and then, it may be determined what quantities of bacteria are required to provide the desired L-AI activity in the dairy product.

The lactic acid fermentation of milk may be intended for the production of any fermented dairy product, for instance yoghurt (stirred style yoghurt, set style yoghurt or yoghurt beverages), cultured buttermilk (skim or whole milk fermented most frequently with *Lactococcus lactis*), Acidophilus milk (i.e. a milk fermented with *Lactobacillus acidophilus),* sour cream (cream inoculated most frequently with *Lactococcus lactis*), and local beverages or products such as kefir, koumiss, beverages based on lactobacillus or bifidus strains, labneh.

In particular, yoghurt is produced by inoculating milk with a starter culture which is generally a symbiotic blend of *Streptococcus thermophilus* (ST) and *Lactobacillus bulgaricus* (LB).

### Method of producing dairy products containing D-tagatose, and edulcorating milk additive

The application discloses a method of converting D-galactose into D-tagatose in a dairy product containing D-galactose, which method comprises the step of adding at least one L-arabinose isomerase and/or one L-arabinose isomerase-expressing microorganism acceptable for food consumption into said dairy product containing D-galactose, whereby D-galactose present in the dairy product is converted into D-tagatose.

The application also discloses a method of converting D-galactose into D-tagatose in a dairy product containing D-galactose, which method comprises the step of contacting said dairy product containing D-galactose with at least one L-arabinose isomerase and/or one L-arabinose isomerase-expressing microorganism, whereby D-galactose present in the dairy product is converted into D-tagatose.

According to the invention the dairy product containing D-galactose is a fermented milk or a milk undergoing lactic acid fermentation and the above methods then consist of methods of converting D-galactose produced by lactic acid fermentation of milk into D-tagatose.

Said method of converting D-galactose produced by lactic acid fermentation of milk into D-tagatose may comprise the steps of :
a) adding into the milk at least one L-arabinose isomerase and/or one L-arabinose isomerase-expressing microorganism acceptable for food consumption;
b) optionally inoculating the milk with one or more lactic acid bacteria; and
c) operating lactic acid fermentation of milk;
whereby D-galactose produced in the milk by lactic acid fermentation is converted into D-tagatose.

The invention further provides a method of converting D-galactose produced by lactic acid fermentation of milk into D-tagatose, which method comprises the steps of:
a) contacting the milk with at least one L-arabinose isomerase and/or one L-arabinose isomerase-expressing microorganism;
b) optionally inoculating the milk with one or more lactic acid bacteria;
c) operating lactic acid fermentation of milk; and
d) optionally recovering said at least one L-arabinose isomerase and/or one L-arabinose isomerase-expressing microorganism from the fermented milk obtained from step c);
whereby D-galactose produced in the milk during lactic acid fermentation is converted into D-tagatose.

Step a) of the above methods of converting D-galactose produced by lactic acid fermentation of milk into D-tagatose may be carried out before, simultaneously or after step c).

Where the method involves addition of, or contacting with, a L-arabinose isomerase-expressing microorganism, if said microorganism is not a lactic acid bacteria, then the method comprises a step of inoculating the milk with at least one lactic acid bacterium so that lactic acid fermentation of milk can be carried out.

Indeed, the presence of lactic acid bacteria in the milk is compulsory in order to operate lactic acid fermentation. If the L-arabinose isomerase-expressing microorganism is a lactic acid bacterium, addition of one or more lactic acid bacteria as recited in step b) is optional.

In the method wherein the L-AI or L-AI expressing microorganism is contacted with the milk to be fermented or undergoing fermentation, step d) is preferably carried out once lactic acid fermentation of milk is completed.

Contacting may be performed as described above, for instance by adding into the dairy product a L-AI or L-AI expressing microorganism trapped in a solid structure, for instance in alginate beads, and by removing the solid structure when D-galactose conversion is essentially complete. Contacting may also be performed by contacting the dairy product with a L-AI or a L-AI-expressing microorganism, immobilised on a solid support such as a column or a reactor.

The milk to be fermented may be e.g. raw milk, milk homogeneized and/or pasteurised, skim milk, a milk supplemented with D-galactose, or milk in which lactose was enzymatically processed into glucose and galactose.In the course of the above method of converting D-galactose into D-tagatose during lactic acid fermentation of milk, a fermented dairy product is obtained which contains D-tagatose. As D-tagatose is an edulcorant (sweetener), the method of converting D-galactose into D-tagatose also results in the production of a sweetened fermented dairy product.

The invention thus further provides a method of producing a sweetened fermented dairy product which comprises the steps of :
a) adding at least one L-arabinose isomerase and/or one L-arabinose isomerase-expressing microorganism acceptable for food consumption into said fermented dairy product; or
b) contacting said dairy product containing D-galactose with at least one L-arabinose isomerase and/or one L-arabinose isomerase-expressing microorganism;
whereby a sweetened fermented dairy product is obtained.

Accordingly, the invention further provides a method of producing a sweetened fermented dairy product which comprises the steps of :
a) adding into a milk at least one L-arabinose isomerase and/or one L-arabinose isomerase-expressing microorganism acceptable in milk fermentation;
b) optionally inoculating the milk with one or more lactic acid bacteria;
c) operating lactic acid fermentation of milk;
whereby a sweetened fermented dairy product is obtained.

Another method of producing a sweetened fermented dairy product may comprise the steps of :
a) contacting milk with at least one L-arabinose isomerase and/or one L-arabinose isomerase-expressing microorganism;
b) optionally inoculating milk with one or more lactic acid bacteria;
c) operating lactic acid fermentation of milk; and
d) optionally recovering said at least one L-arabinose isomerase and/or one L-arabinose isomerase-expressing microorganism from the fermented milk obtained from step c);
whereby a sweetened fermented dairy product is obtained.

Step a) of the above methods of producing a sweetened fermented dairy product may be carried out before, simultaneously or after step c).

Where the method involves addition of, or contacting with, a L-arabinose isomerase-expressing microorganism, if said microorganism is not a lactic acid bacteria, then the method comprises a step of inoculating the milk with at least one lactic acid bacterium so that lactic acid fermentation of milk can be carried out.

In the method wherein the L-AI or L-AI expressing microorganism is contacted with the milk to be fermented or undergoing fermentation, step d) is preferably carried out once lactic acid fermentation of milk is completed.

In the above methods, said microorganism is preferably a bacterium acceptable for food consumption.

The lactic acid bacteria (either expressing L-AI or not) to be used in the methods of converting D-galactose into D-tagatose, or of producing a sweetened fermented dairy product, may be any of the lactic acid bacteria which have been defined above. Furthermore, the L-arabinose isomerase and/or one L-arabinose isomerase-expressing microorganism to be added into the milk are as described above.

The step c) of operating lactic acid fermentation of milk is simply performed by culturing the lactic acid bacteria under conditions and for a time sufficient for lactic acid fermentation to take place. The conditions (temperature, etc) and time of culture will be chosen depending on the type of fermented milk product which is intended to be prepared. D-galactose is produced by lactic acid bacteria during lactic acid fermentation and converted into D-tagatose by L-AI activity.

As described above, the fermented dairy product which is produced may be selected from the group consisting of yoghurt, cultured buttermilk, Acidophilus milk, sour cream, kefir, koumiss, and labneh.

In the end, the L-arabinose isomerase or L-arabinose isomerase-expressing microorganism (in particular a bacterium, and preferably a bacterium acceptable for food consumption) which is added into the milk acts as a sweetening additive during lactic acid fermentation of milk. Accordingly, the invention also relates to a sweetening milk additive which comprises a L-arabinose isomerase and/or a L-arabinose isomerase-expressing microorganism as defined herein. Said additive is in particular intended to be used for the manufacture of fermented dairy products.

The invention will be further described in view of the following Figures and Examples.

### FIGURES

Figure 1: Acidification and production of D-tagatose during the growth of MRS9 in MRS supplemented with galactose. Growth was carried out at 37°C. (A): pH profile, (◆):D-tagatose production with MRS9 strain.
Figure 2: Acidification and production of D-tagatose during the growth MRS12 in M17 supplemented with galactose. Growth was carried out at 37°C. (A): pH profile, (■):D-tagatose production with MRS12 strain.
Figure 3: Production of D-tagatose during the growth at pH 6.4 of MRS9 in MRS supplemented with galactose and MRS12 in M17 supplemented with galactose. Growth was carried out at 37°C. (A): pH profile, (◆):D-tagatose production with MRS9 strain, (■):D-tagatose production with MRS12 strain.
Figure 4: Kinetic of the D-galactose conversion into D-tagatose in milk with D-galactose (10 g/L) and the purified L-AI US100 (3 mg/ml). Incubation was performed at 42 °C.
Figure 5: Temperature activity profile of the purified L-AI US100 and its derivatives. Assays were carried out under standard conditions in presence of 5 mM L-arabinose. Activities at the optimal temperature were defined as 100%.
Figure 6: pH activity profiles of the purified L-AI US100 and its derivatives. Assays were carried out under standard conditions in presence of 5 mM L-arabinose. Activities at the optimal pH were defined as 100%.
Figure 7: Appearance of D-tagatose using beads containing cells expressing the wild type L-AI US100 in milk enriched in D-galactose by lactose hydrolysis.
Figure 8: Appearance of D-tagatose using beads containing cells expressing the mutated L-AI N175H Q268K in milk enriched in D-galactose by lactose hydrolysis.

### EXAMPLES

### Example 1: Bioconversion of D-galactose to D-tagatose in milk, using micro-organisms expressing an L-arabinose isomerase

### 1.1 Material and Methods

### Bacterial strains and growth conditions

*L. bulgaricus* and S. *thermophilus* strains were grown under anaerobic conditions at 42°C or 37°C in MRS medium (Difco) (De Man, Rogosa, and Sharpe. 1960. J. Appl Bacteriol 23:130-135) and M17 lactose medium, respectively (Terzaghi, and Sandine. 1975. Appl Microbiol 29:807-813). These starters were also cultured in skim milk (Nilac Low Heat Milk powder, NIZO) heated for 10 min at 95°C (Courtin, Monnet, and Rul. 2002. Microbiology 148:3413-21). Stock cultures of each strain of S. *thermophilus* and L. *bulgaricus* were prepared after growth at 37°C in skim milk supplemented with antibiotic when required. The cultures were stopped at pH of 5.5, and bacterial numbers were estimated by plating appropriate dilutions of the culture on agar medium: M17Lactose was used for S. *thermophilus* cells, and MRS-Lactose pH 5.2 for *L. bulgaricus* cells. At the end of culture, bacteria were directly frozen in liquid nitrogen and kept at -80°C.

Mixed cultures of wild-type and recombinant S. *thermophilus* and *L. bulgaricus* strains were performed at 42 and 37°C respectively, by inoculating skim milk with 10⁶ c.f.u.ml⁻¹ of stock cultures of each strain. Periodically, the pH of the culture was measured, and bacteria were enumerated as described above. When required, antibiotics were used at the following concentrations: for *E*. *coli,* ampicillin (100 µg/ml) or erythromycin (160 µg/ml); for *L. bulgaricus* and S. *thermophilus,* erythromycin (5 µg/ml). For liquid culture, the recombinant LAB strains were inoculated from an overnight culture at DO_{600 nm} = 0.1 and stopped at DO_{600 nm} = 1.

### DNA manipulations and sequencing

Classical molecular biology techniques and isolation of plasmid DNA from *E. coli* and S. *thermophilus* were performed as described by Sambrook et al. (1989). Plasmid DNA from *L. bulgaricus* was prepared as previously described by Serror et al. (2002. Appl Environ Microbiol 68:46-52). DNA restriction and modifications enzymes were used as recommended by the suppliers. The oligonucleotides used in this work were purchased from PROLIGO (Japan) and MWG (France). When required, DNA fragments were isolated from agarose gels by using the GFX™ PCR DNA and Gel Band Purification Kit (Amersham Bioscience). PCR amplifications, using *Pfu* polymerase (Appligene), were performed with a Gene Amp^{®} PCR System 2700 (Applied Biosystems). Plasmid constructions were transferred from *E. coli* to *L. bulgaricus* VI104 and S. *thermophilus* LMD9 by electroporation (Serror et al. 2002. Appl Environ Microbiol 68:46-52; Somkuti, G. A., and D. H. Steinberg. 1988. Biochimie 70:579-85).

DNA sequencing was performed using an automated 3100 Genetic Analyser (Applied Biosystems).

### Plasmids

All plasmids and strains used in this work are listed in Table 1. The pMR2 plasmid corresponds to pVI165 (an *E. coli*-lactic acid bacteria shuttle, constructed in the laboratory) carrying a 1577 bp Sacll-Sacl DNA fragment, harboring the *araA* US100 gene encoding an L-arabinose isomerase referred to as L-AI US100 without promoter (Rhimi and Bejar. 2006. Biochim Biophys Acta 1760:191-9.). The plasmid pMR3 corresponds to pVI165 carrying a 147 bp fragment containing p_{hlbA}, a strong constitutive promoter from *L. bulgaricus* ATCC11842 (Serror & Chouayekh unpublished, van de Guchte et al, 2006. PNAS). The plasmid pMR4 corresponds to pMR3 carrying the *araA* US100 gene isolated from pMR1 (Sacll - Sacl fragment) downstream of the p_{hlbA} promoter.

**Table 1: Bacterial strains and plasmids**

| **Strains** | **Characteristics** | **Source/reference** |
|---|---|---|
| *Streptococcus thermophilus* LMD9 | Type strain | Bolotin et al. Nat Biotechnol. 2004; 22(12):1554-8. |
| *Lactobacillus delbrueckii* subsp *bulgaricus* ATCC 11842 | Type strain | van de Guchte et al. Proc Natl Acad Sci USA. 2006 103(24):9274-9. |
| *Lactobacillus delbrueckii* subsp. *bulgaricus* VI104 | | Serror et al.. Appl Environ Microbiol. 2002. 68(1):46-52 |
| Plasmids | | |
| pVI165 | pVI1055 (Serror et al. Microbiology. 2003.149:1503-11) containing a cluster of 3 transcription terminators (from pVI152) | (Chouayekh & van de Guchte, unpublished). |
| pMR2 | *araA* US100 (Rhimi & Bejar 2006. BBA **1760**:191-9) cloned in pVI165 | This work |
| pMR3 | p_{hlbA} promoter cloned in pVI165 | This work |
| pMR4 | *araA* US100 from pMR1 cloned in pMR3 downstream the *hlbA* promoter | This work |

### Preparation of crude enzyme extracts

For lactic acid bacteria, cultures done in the appropriate media were centrifuged and the pellets were suspended in 100 mM MOPS Buffer (pH 7.5). However, in skim milk cultures, corresponding to the separate or co-culture of *L. bulgaricus*/pMR4 and S. *thermophilus*/pMR4 strains, before cell centrifugation 0.3 volume of 1 M Tris-sodium citrate was added to the culture. Cells were then centrifuged and cells pellets were washed three times with extraction Buffer (20 mM Sodium phosphate, pH 6.4). Then, recovered pellet was suspended in 100 mM MOPS Buffer (pH 7.5).

Cell disruption was performed using a mini-bead beater 8TM cell disrupter, and cell debris were removed by centrifugation at 15000 rpm for 30 min at 4°C.

### Protein quantification, electrophoresis and identification by mass spectrometry

Protein concentration was determined by the Bradford method (Bradford. 1976. Anal. Biochem 72:248-254). The protein samples were separated in 12 % SDS-PAGE gel electrophoresis according to the method of Laemmli (Laemmli. 1970. Nature 227:680-5). Protein bands were visualized by Coomassie blue Biosafe (BioRad) staining.

Extracted proteins, corresponding to each recombinant strain, were separated on SDS-PAGE electrophoresis. After coloration with Coomassie blue Biosafe staining (BioRad), bands having the expected molecular weight were excised from the gel and washed in acetonitrile/50 mmol.l⁻¹ ammonium hydrogenocarbonate (v/v). Then, samples were stirred for 15 min, vacuum-dried for 30 min, and 0.5 mg trypsin (Promega) in 25 ml ammonium hydrogenocarbonate (50 mmol.l⁻¹) was added. Samples were digested for 16 h at 37°C. For Matrix Assisted Laser Desorption lorization-Time of Flight (MALDI-TOF) analyses, 2 µl TFA 5 % was added to stop the trypsinolysis reaction. Peptides mixtures were analyzed using a cyano-4-hydroxycinamic matrix prepared at 5 mg/ml in 50 % acetonitrile containing 0.1 % trifluoroacetic acid. The trypsin autolytic peptides (842.5 and 2211.1 Da) were used as internal calibrants. Peptides were selected in the mass range 700-4000 Da.

Peptides were analysed using a Voyager DE STR MALDI-TOF mass spectrometer (Applied Biosystems). Recorded MS/MS spectra were compared to theoretical fragmentations of a trypsinolysed araA US100 protein (Accession number: CAI29261), via the Sequest algorithm incorporated in the BioWorks software (version 2, Thermo Finnigan).

### Assay for L-arabinose isomerase activity

L-arabinose isomerase activity was measured by the determination of the amount of the produced L-ribulose or D-tagatose. In standard conditions, the reaction mixture contained 0.2 mM CoCl₂, 1 mM MnSO₄, 50 □µl of crude enzyme extract at a suitable dilution, 5 mM of L-arabinose (D-galactose) and MOPS buffer 100 mM (pH 7.5) to bring the final volume to 1 ml. The reaction mixture was incubated at 80°C for 1 min and 10 min for L-arabinose and D-galactose respectively. Then, samples were immediately chilled on ice to stop the reactions. The generated L-ribulose (or D-tagatose) was determined by the cysteine carbazole sulfuric-acid method, and the absorbance was measured at 560 nm (Dische and Borenfreund. 1951. J Biol Chem 192:583-7). The D-tagatose production was also checked with high-performance ionic chromatography (HPLC) using Polypore CA column (250 × 4.6 mm). The products were separated by isocratic elution with water at a flow rate of 0.3 ml/min and detected with a refractive index detector (SHIMADZU, Refractive Index Detector RID-10A). A 10 g/l solution of lactose, D-galactose and D-tagatose were used as standards. One unit of L-arabinose isomerase activity was defined as the amount of enzyme catalyzing the formation of 1 µmol keto-sugar per min under the above specified conditions.

### Detection of in vivo D-tagatose production conferred by L-AI US100 expression

The *L. bulgaricus* and S. *thermophilus* strains containing pMR4 were cultured in MRS and M17 media containing D-galactose at final concentration of 10 g/l, respectively. Using an overnight culture, the medium was inoculated at DO_{600 nm} of 0.1. Samples were collected each hour, until 12 hours, followed by the monitoring of the L-AI US100 activity by cysteine carbazole sulfuric-acid method in the supernatant. The D-tagatose production was also checked HPLC.

### Detection of the in vitro D-tagatose production in milk using L-AI US100

The purified L-AI US100 (3 mg/ml) was added in milk supplemented with D-galactose (final concentration of 10 g/l). Alternatively, the purified enzyme was added to the co-culture of the wild-type and recombinant *L. bulgaricus* and *S*. *thermophilus* strains performed in milk. The study of the kinetic bioconversion of D-galactose was investigated until 20 hours. Milk proteins were removed by defecation step which consists of the addition of one volume of 30 % Zinc acetate and 15 % potassium Ferrocyanure (w/v) solutions respectively, followed by a centrifugation at 15000 during 20 min. Then, sugars were recovered in the aqueous phase. The D-tagatose production, in this supernatant fraction, was proved via cysteine carbazole sulfuric-acid method and confirmed by HPLC.

### 1.2 Results

### Production of active L-AI US 100 in/from yoghurt starters

The plasmid pMR4 containing p_{hlbA}:*araA* US100 fusion and the two negative controls pMR2 (containing the *araA* gene without promoter) and pMR3 (containing p_{hlbA} alone) were introduced in the *L. bulgaricus* (strain VI104) and *S*. *thermophilus* (strain LMD9) yoghurt starters, which do not metabolize galactose during lactose fermentation. Expression of L-AI activity was checked in crude extracts of the resulting strains grown in synthetic and milk media using cysteine carbazole sulfuric-acid method (Material and methods).

After growth in synthetic media, the L-AI activity was only detected in the crude cellular extracts prepared from the *L. bulgaricus* and S. *thermophilus* strains containing pMR4 (p_{hlbA}:*araA* US100), i.e. MRS9 and MRS12, respectively. The activities measured were 25±0.32 and 15±0.7 U/mg for *L. bulgaricus* and S. *thermophilus,* respectively (Table 2).

**Table 2: L-AI US100 activity expressed by the L. bulgaricus and S. thermophilus strains grown in synthetic or milk media.**

| **Strain description** | **Strain designation** | **L-AI US100 activity (U/mg) Laboratory medium** | **L-AI US100 activity (U/mg) Milk** |
|---|---|---|---|
| VI104/pMR2 | MRS7 | ND | ND |
| VI104/pMR3 | MRS8 | ND | ND |
| VI104/pMR4 | MRS9 | 25±0.32 | 21±0.14 |
| LMD9/pMR2 | MRS10 | ND | ND |
| LMD9/pMR3 | MRS11 | ND | ND |
| LMD9/pMR4 | MRS12 | 15±0.7 | 12±0.18 |
| VI104/pMR4 & LMD9/pMR4 | MRS9 and MRS12 | ND | 32±0.54 |

| | | | |
|---|---|---|---|
| ND: Not Detected | | | |

In addition to the measurement of the L-AI activity *in vitro,* crude cellular extracts prepared from *L. bulgaricus* and *S. thermophilus* harboring the various plasmids pMR2, pMR3 and pMR4 were run on SDS-PAGE and stained with Coomassie blue. At the molecular weight expected for the L-AI US100 monomer (56 kDa), new bands were detected in the samples corresponding to the *L. bulgaricus* and S. *thermophilus* strains carrying pMR4. These bands as well as their weaker counterparts in the control samples were excised and proteins were analyzed by mass spectrometry (MS). L-AI US100 was unambiguously identified in the bands originating from the strains containing pMR4 whereas it was not detected in the thinner bands obtained with negative controls.

Subsequently, the expression and functionality of L-AI US100 were evaluated in milk, the usual growth condition of yoghurt starters. *L. bulgaricus* and S. *thermophilus* strains carrying pMR4 (p_{hlbA}:*araA*) were cultivated separately or in association in milk and the LAI activity was measured in crude cellular extracts. In single species cultures, the *in vitro* L-AI specific activities were 21±0.14 and 12±0.18 U/mg for *L. bulgaricus* and S. *thermophilus* strains carrying pMR4, respectively. In the co-culture, the L-AI specific activity reproducibly reached 32±0.54 U/mg (Table 2). An analysis using SDS-Page electrophoresis and mass spectrometry confirmed the presence of L-AI US100 only in the extracts derived from the milk cultures of strains containing pMR4.

These observations were in good agreement with the *in vitro* L-AI activity measurements that clearly proved the efficient expression of L-AI US100 in both yoghurt starter species under the control of the p*_{hlbA}* promoter.

### Bioconversion of D-galactose into D-tagatose by strains expressing the L-AI US100 in medium enriched with D-galactose

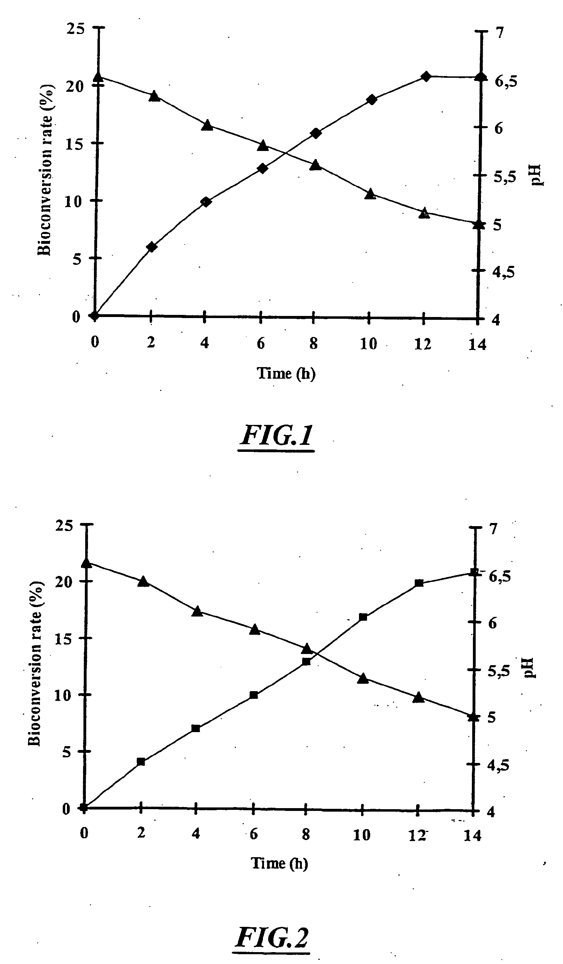

To test the D-galactose isomerisation by the *in vivo* expressed L-AI US100, the L. *bulgaricus* and S. *thermophilus* recombinant strains were grown in laboratory media enriched with 10 g/l of D-galactose. Kinetic studies during the growth of *L. bulgaricus* and S. *thermophilus* carrying pMR4 revealed that a new ketohexose was formed in the culture medium (Figures 1-2). This ketohexose identified as D-tagatose by HPLC, accumulated up to 20% of the overall ketohexose (i.e. about 2 g/L) after 12 hours of fermentation.

### Bioconversion of D-galactose into D-tagatose during milk fermentation

The *in vivo* bioconversion of the D-galactose produced during milk fermentation by means of the LAB starters expressing *araA* (MRS9 and MRS12) was then tested. A sample fermented by the parental LAB starters which did not exhibit any L-AI activity was also used as a control. The sugar composition of the growth medium was determined by HPLC at various incubation times at 42°C (Table 3). D-galactose accumulated in all cultures but D-tagatose was only identified in the cultures supplemented with L-AI via its expression from the LAB starters.

**Table 3: Analysis of sugar composition in milk fermented by various L. bulgaricus and S. thermophilus starters.**

| | Time (h) | VI104 & LMD9 | MRS9 & MRS12 |
|---|---|---|---|
| D-galactose (g/l) | 0 | 0 | 0 |
| | 10 | 6.6 | 3.9 |
| | 20 | 10.2 | 6.2 |
| D- tagatose (g/l) | 0 | 0 | 0 |
| | 10 | 0 | 0.33 |
| | 20 | 0 | 0.72 |

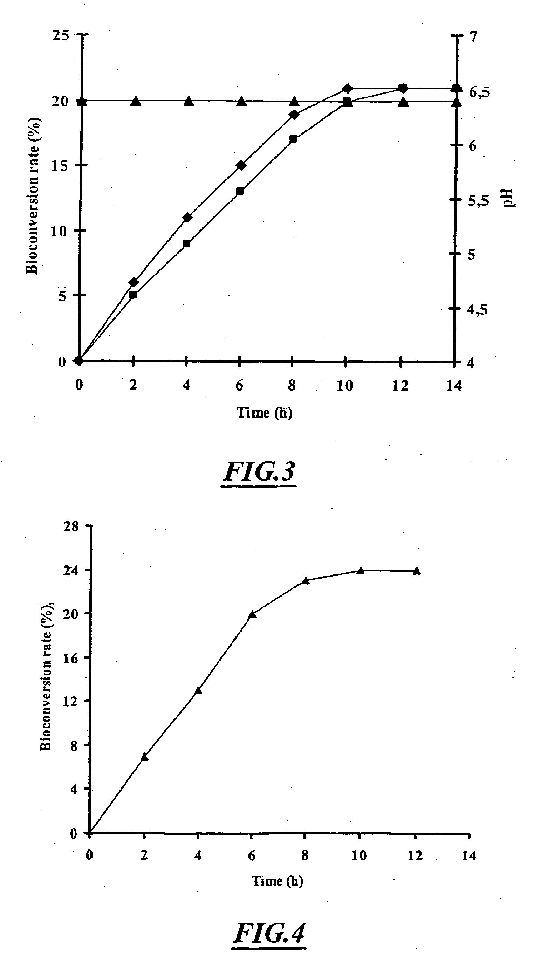

Considering that the optimal pH for L-AI activity is about pH 7.5-8, it was wondered whether the acidification of the medium resulting from the production of lactic acid by the LAB starters (Figures 1-2) could impair the efficiency of the expressed L-AI and the kinetic of D-tagatose conversion. In order to investigate the effect of the medium acidification on the yield of D-tagatose production, MRS9 and MRS12 strains were grown in a bioreactor allowing the neutralization of lactic acid by the addition of NaOH (5N) (Figure 3). In this system, the final bioconversion yield was similar but the kinetic of apparition of D-tagatose was much faster: the maximal amount of D-tagatose was reached after 9 h instead of 12 h.

All these results, established that the L-AI US100 enzyme produced by the LAB strains is active and allows the conversion of D-galactose in medium enriched with D-galactose and during milk fermentation although the enzyme is in the bacterial cytoplasm.

*Conversion of D-galactose by supernatant of LAB secreting the* L-AI *US100* The L: strain MG1363 containing the plasmid *pSEC:araUS100* was used to expressed the *ara* gene fused to a sequence encoding the peptide signal of Usp45, the main secreted protein of *L. lactis.* The control strain is an isogenous strain containing the same plasmid deleted of the secretion signal. The L-AI activity was detected in the supernatant of the culture MG1363/pSec:*araUS100* indicating that the enzyme is secreted when associated to a functional peptide signal. The supernatant was added to synthetic growth medium and milk supplemented with D-galactose (10 g/L). The bio-conversion of galactose in D-tagatose was observed in both media albeit at low efficiency; after 10 h of incubation, 0.4 g/L of D-tagatose was measured in each of the samples. This observation established for the first time that the L-AI enzyme can be secreted into the medium when its gene is fused to a peptide signal encoding sequence and is secreted in its active form in the medium. This mode of enzyme production may be interesting for industrial preparation of the enzyme after improvement of the efficiency of expression-secretion.

### Example 2: Production of D-tagatose by a L-arabinose isomerase used as an additive in milk enriched in D-galactose

### 2.1 Material and Methods

### Bacterial strains and growth conditions

The bacterial strain used to purify the L-AI US100 for this work is HB101 containing pMR6, a pTRC99A derivative carrying the *araA* gene of *B. stearothermophilus* US100. This *E. coli* strain was grown in aerated Luria-Bertani medium (Difco) at 37°C (Sambrook et al.. 1989. Molecular cloning: a laboratory manual, 2nd ed. Cold Spring Harbor Laboratory Press, Cold Spring Harbor, New York, USA). *L. bulgaricus* and S. *thermophilus* strains were cultured in skim milk (Nilac Low Heat Milk powder, NIZO) heated for 10 min at 95°C (Courtin, Monnet, and Rul 2002. Microbiology 148:3413-21). Stock cultures of each strain of S. *thermophilus* and *L. bulgaricus* were prepared after growth at 37°C in skim milk supplemented with antibiotic when required. The cultures were stopped at pH of 5.5, and bacterial numbers were estimated by plating appropriate dilutions of the culture on agar medium: M17Lactose was used for S. *thermophilus* cells, and MRS-Lactose pH 5.2 for *L. bulgaricus* cells. At the end of culture, bacteria were directly frozen in liquid nitrogen and kept at -80°C. Mixed cultures of S. *thermophilus* and *L. bulgaricus* strains were performed at 42°C by inoculating skim milk with 10⁶ c.f.u/ml⁻¹ of stock cultures of each strain. Periodically, the pH of the culture was measured, and bacteria were enumerated as described above.

### Preparation of crude extracts and protein purification

Cells were harvested by centrifugation at (7500 × g, for 10 min) and the pellets were suspended in 100 mM HEPES buffer supplemented, where indicated, with 1 mM MnCl₂ and 0.2 mM CoCl₂. Then, cell suspensions were incubated for two hours on ice in the presence of 5 mg/ml lysozyme, 100 mM PMSF (phenylmethane-sulfonyl fluoride) and 2 µg/ml pepstatin A. Cell disruption was carried out by sonication at 4°C for 6 min (pulsations of 3 s, amplify 90) using a Vibra-Cell™ 72405 Sonicator and cell debris were removed by centrifugation (30 000×g, for 30 min at 4°C).
For protein purification, crude cell extract from each strain was heated (70°C for 30 min) followed by centrifugation at 30 00×g, for 30 min at 4°C. Proteins were precipitated between 60 and 80 % ammonium sulphate saturation, suspended in 100 mM HEPES buffer pH 7.5, concentrated and desalted in centrifugal micro-concentrators (Amicon, Inc) with a 30 kDa membrane cut off. Purification to homogeneity was achieved by fast-performance liquid chromatography (FPLC-Amersham-Bioscience) using a UNO-Q anion-exchange column equilibrated with 100 mM HEPES buffer (pH 7.5). The proteins were eluted at a flow rate of 3 ml/min using a linear NaCl gradient ranging from 0 to 1 M in the same HEPES buffer. Pooled fractions displaying L-AI activity were desalted and concentrated.

### Assay for L-arabinose isomerase activity

L-arabinose isomerase activity was measured by the determination of the amount of the produced L-ribulose or D-tagatose. In standard conditions, the reaction mixture contained 0.2 mM CoCl₂, 1 mM MnSO₄, 50□ µl of crude enzyme extract at a suitable dilution, 5 mM of L-arabinose (D-galactose) and MOPS buffer 100 mM (pH 7.5) to bring the final volume to 1 ml. The reaction mixture was incubated at 80°C for 1 min and 10 min for L-arabinose and D-galactose respectively. Then, samples were immediately chilled on ice to stop the reactions. The generated L-ribulose (or D-tagatose) was determined by the cysteine carbazole sulfuric-acid method, and the absorbance was measured at 560 nm (Dische and Borenfreund. 1951. J Biol Chem 192:583-7). The D-tagatose production was also checked with high-performance ionic chromatography (HPLC) using Polypore CA column (250 × 4.6 mm). The products were separated by isocratic elution with water at a flow rate of 0.3 ml/min and detected with a refractive index detector (SHIMADZU, Refractive Index Detector RID-10A). A 10 g/l solution of lactose, D-galactose and D-tagatose were used as standards. One unit of L-arabinose isomerase activity was defined as the amount of enzyme catalyzing the formation of 1 µmol keto-sugar per min under the above specified conditions.

### Detection of the in vitro D-tagatose production in milk using L-AI US100

The purified L-AI US100 (3 mg/ml) was added in milk supplemented with D-galactose (final concentration of 10 g/l). Alternatively, the purified enzyme was added to the co-culture of the wild-type and recombinant *L. bulgaricus* and S. *thermophilus* strains done in milk. The study of the kinetic bioconversion of D-galactose was investigated until 20 hours. Milk proteins were removed by defecation step which consists on the addition of one volume of 30 % Zinc acetate and 15 % potassium Ferrocyanure (w/v) solutions respectively, followed by a centrifugation at 15000 during 20 min. Then, sugars were recovered in the aqueous phase. The D-tagatose production, in this supernatant fraction, was proved via cysteine carbazole sulfuric-acid method and confirmed by HPLC.

### 2.2 Results

### Isomerisation of D-galactose to D-tagatose by the addition of L-AI US100 in milk enriched with D-galactose

The possibility to add the L-AI US100 enzyme directly in milk to promote the isomerisation of D-galactose into D-tagatose was tested. As a first step, the purified enzyme was added to milk supplemented with D-galactose and incubated at 42°C. The kinetic of D-tagatose production was followed using the cysteine carbazole sulfuric-acid method. The bioconversion of D-galactose increased proportionally with the incubation period and a maximal conversion rate of 24 % was reached after nearly 8 hours of incubation (Figure 4). The conversion of D-galactose into D-tagatose was confirmed by HPLC analysis, which showed a decrease in D-galactose concentration concomitant with an increase in D-tagatose production. These results and comparison with the activity measured in a standard reaction performed at 42°C aid pH 6.5 (not shown) evidenced that L-AI US100 enzyme was fully active when used as an additive in milk.

### Isomerisation of D-galactose produced during milk fermentation by the addition of L-AI US100

The *in vivo* bioconversion of the D-galactose produced during milk fermentation by means of the L-AI US100 enzyme was then tested. The milk was fermented by LAB starters in presence of 1 mg/ mL of purified L-AI enzyme used as an additive. Analysis of sugar composition of the fermentation medium showed an accumulation of D-tagatose. The sugar composition of the growth medium was determined by HPLC at various time of the fermentation (Table 4). D-galactose accumulated in all cultures but D-tagatose was only identified in the cultures supplemented with L-AI by the addition of purified enzyme.

**Table 4: Analysis of sugar composition in milk fermented by LAB with L-AI US100. LAB starter is L. bulgaricus VI104 and S. thermophilus LMD9.**

| | Time (h) | Starter strains only | Starter and enzyme (mg/ml) | | | |
|---|---|---|---|---|---|---|
| | | | 1 | 2 | 3 | 4 |
| D- tagatose (g/l) | 0 | 0 | 0 | 0 | 0 | 0 |
| | 4 | 0 | 0.3 | 0.5 | 0.6 | 0.5 |
| | 6 | 0 | 0.6 | 0.8 | 0.9 | 0.7 |
| | 10 | 0 | 1.5 | 1.7 | 2.1 | 1.7 |
| | 20 | 0 | 2.3 | 2.3 | 2.7 | 2.3 |

### Optimisation of the enzyme concentration needed for the D-tagatose production during milk fermentation.

D-tagatose production by adding the purified L-AI US100 was explored. In fact, the concentration of the added protein in culture was varied between 1 and 4 mg/ml. The results obtained indicate that the kinetic D-galactose conversion increased proportionally to the protein concentration until 3 mg/l. Above this concentration, the addition of higher enzyme concentrations did not lead to a noteworthy effect (Table 4).

Altogether, these results establish for the first time that an L-arabinose isomerase used as an additive during milk fermentation efficiently converts the D-galactose produced by the fermentation in D-galactose.

### Example 3: Bioconversion of D-galactose to D-tagatose in milk by L-arabinose isomerase derivatives

### 3.1 Materials and Methods

### Bacterial strains, media, plasmids and growth conditions

*Escherichia coli* HB101 (F *hsdS20 ara-1 recA13 proA12 lacY1 galK2 rpsL20 mtl-1 oxy*/*-5*) was used as an *araA*-deficient host strain for the purification of the L-arabinose isomerase and its derivatives. Culture of *E*. *coli* strains, harbouring wild-type and mutated gene, was done in Luria Bertani (LB) medium (Sambrook et al., 1989 supra) and supplemented, when necessary, with ampicillin (100 µg/ml) and IPTG (160 µg/ml). The plasmid pMR5 containing the wild-type *araA* US100 gene (Rhimi and Bejar, Biochim Biophys Acta 1760 (2006) 191), was used as template and for the production of the wild-type L-AI US100 protein. Plasmid pMR37 carries the *araA* S71 P gene, pMR35 carries the *araA* N175H gene, pMR21 contains the *araA* Q268K gene and pMR36 carries the *araA* N175H Q268K gene.

### PCR and DNA manipulation

Polymerase chain reactions were carried out in Gene Amp^{®} PCR System 9700 (Applied Biosystems). The amplification reaction mixtures (50 µl) contained *pfu* (Turbo DNA polymerase) amplification buffer, 10 mM (NH₄)₂SO₄, 100 ng of each primer, 50 ng of DNA template, and 2 Units of *pfu* enzyme (Appligene). The cycling parameters were 94°C for 5 min, followed by 40 cycles at 94°C for 30 s, 60°C for 60 s, and 72°C for 120 s. Preparation of plasmid DNA, digestion with restriction endonucleases and separation of fragments by agarose gel electrophoresis were performed as described by Sambrook et al. (1989, supra).

### Construction of L-arabinose isomerase derivatives

The L-arabinose isomerase derivatives were all generated by site-directed PCR mutagenesis (as described below for mutation N175H and Q268K and with the Quick change II XL-site directed mutagenesis Kit [Stratagene] for S71 P and the double mutant N175H Q268K) using the wild-type L-AI US100 coding sequence in plasmid pMR5 as template. Thus, two non-mutagenic external primers F-araA and R-araA, and for each derivatives two complementary internal primers containing the desired mutation were designed (Table 5).

**Table 5: Oligonucleotides used for site directed mutagenesis. Nucleotide sequences corresponding to the mutated amino-acids are underlined.**

| Desiqnation | Primer set |
|---|---|
| FaraA | GTGAACGGGGAGGAGCAATG (SEQ ID NO :3) |
| RaraA | GAAATCTTACCGCCCCCGCC (SEQ ID NO :4) |
| S71P D | GAATGGAATCGCGATCCGGTGTTCCGTTCCCA (SEQ ID NO :7) |
| S71 P R | TGGGAACGAAACACCGGATCGCGATTCCATTC (SEQ ID NO :8) |
| N175H D | GCGTTTGCGGAAAGCCGCAACCTAAAAGTGGCTCGT (SEQ ID NO :9) |
| N175H R | ACGAGCCACTTTTAGGTTGCGGCTTTCCGCAAACGC (SEQ ID NO :10) |
| Q268K D | GCCTTTTTGAAAGACGGGAAC (SEQ ID NO :5) |
| Q268K R | GTTCCCGTCTTTCAAAAAGGC (SEQ ID NO :6) |

Then, two separate PCR reactions were done and the resulting fragments were amplified using pMR5 as template with the corresponding primers for each reaction. The resulting two PCR fragments were extracted separately, and then a third amplification was carried out on a mixture containing these fragments in the presence of the external primers. The PCR products were purified using the GFX™ PCR DNA and Gel Band Purification Kit (Amersham Bioscience), following the manufacturer's instructions.

The purified mutated DNA fragments were cloned into a pUT57 vector, previously linearized with the *Sma*I restriction enzyme, and transformed into the HB101 strain. Transformants were plated on LB medium supplemented with ampicillin (100 µg/ml) and IPTG (160 µg/ml). The obtained colonies were analysed by restriction and mutations were confirmed by DNA sequencing using an automated DNA sequencer (Applied Biosystems).

### Preparation of crude extracts and protein purification

Cells were harvested by centrifugation at (7500 × g, for 10 min) and the pellets were suspended in 100 mM HEPES buffer supplemented, where indicated, with 1 mM MnCl₂ and 0.2 mM CoCl₂. Then, cell suspensions were incubated for two hours on ice in the presence of 5 mg/ml lysozyme, 100 mM PMSF (phenylmethane-sulfonyl fluoride) and 2 µg/ml pepstatin A. Cell disruption was carried out by sonication at 4°C for 6 min (pulsations of 3 s, amplify 90) using a Vibra-Cell™ 72405 Sonicator and cell debris were removed by centrifugation (30 000×g, for 30 min at 4°C).

For protein purification, crude cell extract from each strain was heated (70°C for 30 min) followed by centrifugation at 30 000×g, for 30 min at 4°C. Proteins were precipitated between 60 and 80 % ammonium sulphate saturation, suspended in 100 mM HEPES buffer pH 7.5, concentrated and desalted in centrifugal micro-concentrators (Amicon, Inc) with a 30 kDa membrane cut off. Purification to homogeneity was achieved by fast-performance liquid chromatography (FPLC-Amersham-Bioscience) using a UNO-Q anion-exchange column equilibrated with 100 mM HEPES buffer (pH 7.5). The proteins were eluted at a flow rate of 3 ml/min using a linear NaCl gradient ranging from 0 to 1 M in the same HEPES buffer. Pooled fractions displaying L-AI activity were desalted and concentrated.

### Protein quantification and electrophoresis

Protein concentrations were determined using Bradford's method with bovine serum albumin as standard (Bradford. Anal Biochem 72 (1976) 248). The purified enzyme samples were migrated in 12% sodium dodecyl sulfate-polyacrylamide gel electrophoresis (SDS-PAGE) according to the method of Laemmli (Laemmli.1970. Nature 227:680). Protein bands were visualized by Coomassie brilliant blue R-250 (BioRad) staining.

### Enzyme assays and determination of the temperature and pH effects

L-arabinose isomerase activity was measured by determining the amount of L-ribulose or D-tagatose formed in the following reaction. The standard reaction mixture contained 0.2 mM CoCl₂, 1 mM MnCl₂, 50□ µl of enzyme preparation at a suitable dilution, 5 mM of L-arabinose or D-galactose and MOPS buffer 100 mM (pH 7.5) to bring the final volume to 1 ml. The reaction mixture was incubated at 80°C for 1 min and 10 min for L-arabinose and D-galactose, respectively, followed by cooling of the samples on ice to stop the reaction. The generated L-ribulose (or D-tagatose) was determined by the cysteine carbazole sulfuric-acid method, and the absorbance was measured at 560 nm (Dische and Borenfreund, J Biol Chem 192 (1951) 583). The D-tagatose production was also confirmed by high-performance ionic chromatography (HPLC) using a Polypore CA column (250 × 4.6 mm). The products were separated by isocratic elution with water at a flow rate of 0.3 ml/min and detected with a refractive index detector (SHIMADZU, Refractive Index Detector RID-10A). Solutions 5 g /l of D-galactose and D-tagatose were used as standards. The thermoactivity profile was studied by performing the standard reaction (described above) at temperatures comprised between 30 and 90°C. The pH effect was measured by carrying out the standard reaction (described above) at pHs ranging from 4.0 to 9.0 (4 to 6 adjusted with acetate, 6.0 to 8.0 using MOPS buffer and 8.0 to 9.0 using Glycine buffer).

### 3.2. Results

### Mutant design, purification and biochemical characterization

The optimal conditions for L-AI US100 activity (80°C and pH 7.5) may not be always adapted to the conditions of food processing e.g., in the process of milk fermentation by LAB starters (42°C to 44°C and pH between 6.5 and 4.5). Therefore, the biochemical properties of various L-AI derivatives were investigated. The catalytic residues implicated in the isomerisation reaction catalysed by L-AI US100, and those involved in the enzyme affinity were previously identified (Rhimi et al., J Bacteriol. 2007. 189(9):3556-63). In order to investigate the relationship between the structure and the function of the L-AI US100, several derivatives of this enzyme were generated by site-directed PCR mutagenesis. The activity of several of these L-AI derivatives was tested at various temperatures and pH.

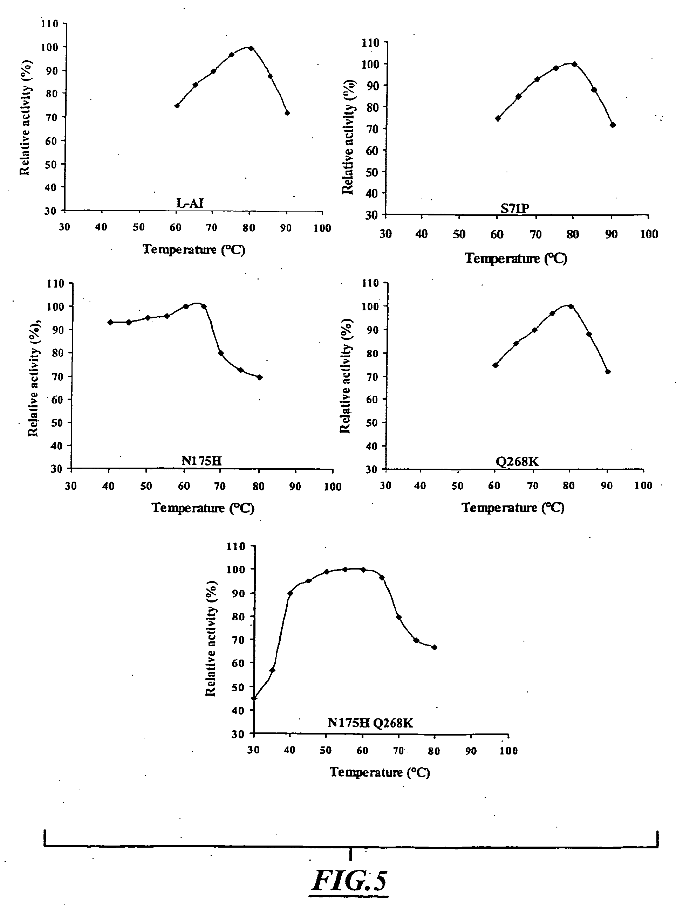

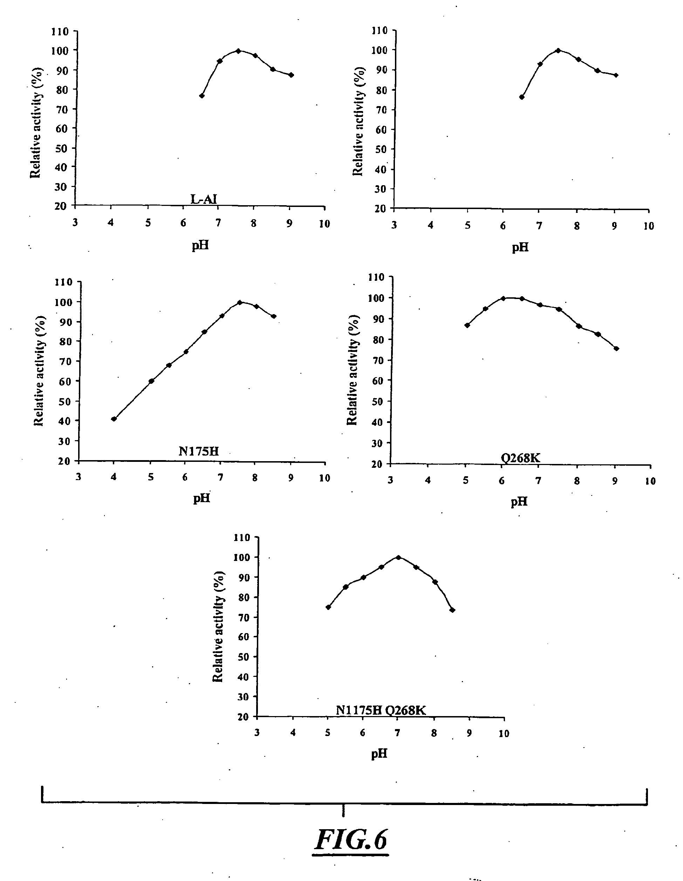

The activity study at different temperatures showed that the L-AI US100 protein, the L-AI US100 S71 P and the L-AI US100 Q268K had similar profiles and were optimally active at 80°C. In contrast, L-AI US100 N175H and the double mutant L-AI US100 N175H Q268K exhibited different patterns with optimal temperatures of 60-70°C and 55-60°C, respectively (Figure 5). Regarding the optimal pH for activity, L-AI US100 and its derivatives L-AI US100 S71P and L-AI US100 N175H similarly displayed the highest activity at pH ranging between 7.5 and 8.0, whereas other derivatives such as the L-AI US100 Q268K and the double mutant L-AI US 100 N175H Q268K had an optimal pH of 6.0-6.5 and 7.0, respectively (Figure 6). In addition thereto, the relative activity values of the mutated L-AI N175H Q268K and L-AI Q268K were increased from pH 5.0 to 6.5 by more than 15% and by 25 % as compared to those of wild-type L-AI US100, respectively.

These results demonstrate that the N175H mutation shifted the enzyme optimal temperature from 80°C to 60-70°C without affectingthe enzyme optimal pH while the Q268K mutation significantly shifted the optimal pH of the mutated enzyme from 7.5-8, as observed for the wild-type L-AI US100, to 6.0-6.5 while it did not change the enzyme optimal temperature. In other words, the N175H mutation improved the mesophily of the enzyme while the Q268K mutation increased its acidotolerance. The effect of these mutations cumulates in the L-AI US100 N175H Q268K derivative which is both more mesophile and more acidotolerant than the wild-type L-AI US100 enzyme. Although the mutation S71P had no effect on the two parameters tested above, it (as other L-AI mutations) may prove beneficial for other biochemical parameters and for given applications.

### D-tagatose production by L-AI derivatives in milk enriched with D-galactose

The conversion of D-galactose (10 g/L) in milk was tested with four of the L-AI US100 derivatives (3 mg/mL). At various time during the incubation at 42°C, samples were harvested and the amount of D-tagatose produced was measured (Table 6). Although at 20h, the final conversion yield was similar for all enzymes, the production of D-tagatose was faster in the samples containing L-AI N175H Q268K or L-AI N175H than in samples with L-AI Q268K or L-AI US100. This observation is in complete agreement with the biochemical properties of the L-AI derivatives. The mesophily of L-AI N175H and L-AI N175H Q268K indeed allow these enzymes to be more active in the condition tested i.e., pH6.5 and 42°C than L-AI US100 and the aciddolerant L-AI Q268K.

**Table 6: production of D-tagatose by L-AI US100 and four of its derivatives in milk enriched with D-galactose.**

| **Time (h)** | **L-AI** | **S71P** | **N175H** | **Q268K** | **N175H Q268K** |
|---|---|---|---|---|---|
| 0 | 0 | 0 | 0 | 0 | 0 |
| 4 | 0.6 | 0.6 | 0.9 | 0.6 | 1.0 |
| 6 | 0.9 | 0.9 | 1.3 | 1.1 | 1.7 |
| 8 | 1.0 | 1.0 | 1.7 | 1.4 | 2.0 |
| 10 | 1.1 | 1.1 | 1.9 | 1.7 | 2.1 |
| 20 | 2.3 | 2.3 | 2.4 | 2.3 | 2.4 |

### D-tagatose production by L-AI derivatives during milk fermentation by LAB

The conversion of D-galactose in D-tagatose by L-AI US100 and each of its four derivatives (S71P, Q268K, N175H, N175H Q268K) was then tested during milk fermentation by LAB starters, *L. bulgaricus* VI104 and S. *thermophilus* LMD9. At various fermentation times, samples were harvested to measure the amount of D-tagatose produced and the pH (Table 7). Although at 20 h, the final conversion yield was similar for all enzymes, the production of D-tagatose was faster in samples containing L-AI N175H, L-AI N175H Q268K or L-AI Q268K than in samples with L-AI US100 or L-AI S71P. The conversion of D-galactose appeared faster with L-AI N175H (more mesophile) than with L-AI Q268K (more acidotolerant) but the best conversion rate was observed with L-AI N175H Q268K, the L-AI double mutant which exhibited both a lower optimum temperature and a lower optimum pH for activity compared to L-AI US100. These data demonstrate that modifications of biochemical properties of at least three L-AI derivatives improved their performance during the specific process such as the production of yoghurt or fermented milk.

**Table 7: production of D-tagatose (Tag., in g/L) by various L-AI during milk fermentation.**

| Time (h) | L-AI | | S71P | | N175H | | Q268K | | N175H Q268K | |
|---|---|---|---|---|---|---|---|---|---|---|
| | pH | Tag. | pH | Tag. | pH | Tag. | pH | Tag. | pH | Tag. |
| 0 | 6.6 | 0 | 6.6 | 0 | 6.6 | 0 | 6.6 | 0 | 6.6 | 0 |
| 4 | 5.7 | 0.5 | 5.7 | 0.5 | 5.7 | 0.8 | 5.7 | 0.7 | 5.8 | 1.1 |
| 8 | 5.4 | 0.7 | 5.4 | 0.7 | 5.4 | 1.2 | 5.3 | 1.0 | 5.5 | 1.6 |
| 6 | 5 | 0.9 | 5 | 0.9 | 5 | 1.7 | 4.9 | 1.5 | 5.1 | 1.9 |
| 10 | 4.9 | 1.2 | 4.9 | 1.2 | 5 | 2.1 | 4.8 | 2.0 | 4.8 | 2.4 |
| 20 | 4.9 | 2.5 | 4.9 | 2.5 | 4.9 | 2.5 | 4.8 | 2.5 | 4.7 | 2.5 |

Altogether, construction, purification and use of new L-AI US100 derivatives are described herein. Simple biochemical characterization showed that some mutants have optimum pH and temperature that differs from that of the wild-type L-AI US100. In agreement with these observations, experiments in milk confirmed that these L-AI US100 derivatives can be more efficient for the production of D-tagatose than the genuine enzyme. This work is at the same time a first example, and a clear demonstration that engineering of the L-AI enzyme using random or targeted mutagenesis can allow generating useful L-AI derivatives better suited for the conditions of a given industrial process and therefore very promising for industrial application.

### Example 4: Bioconversion by L-arabinose isomerase in milk enriched in galactose by enzymatic hydrolysis of lactose

### 4.1 Materials and Methods

### Strains

In this example two *E. coli* strains were used. In the strain HB101 containing the pMR4:*araUS100* plasmid, the wild-type *araA US100* gene is under the control of the constitutive *phlbA* promoter. The other strain corresponds to HB101 containing a mutated *araA* gene encoding the double mutant N175H Q268K, under the control of an IPTG-inducible promoter (plac) in the pUT57 plasmid.

### Immobilisation of the micro-organisms and of the enzymes.

Culture of the *E. coli* strains were grown until OD at 600 nm of 3. Cells were then centrifuged and the supernatant discarded. After a drying step the pellet was weighted and suspended in MOPS buffer (pH7.5, 100 mM). Alginate was then added to the cell suspension to obtain the final concentrations of 30 g/l for cells and of 3% for alginate. Cells-containing alginate beads were then formed as droplets.

### Lactose hydrolysis in milk.

The natural lactose of a milk sample was hydrolysed by the addition of Beta-galactosidase originated from *Lactobacillus bulgaricus* ATCC11842. The kinetic od D-glucose apparition was monitored during time; at 25h of incubation, 25 g/l of D-glucose was obtained. As lactose cleavage lead to equimolar amounts of glucose and galactose, we deduced from this measure that the amount of D-galactose also equals to 25 g/L

### 4.2. Results

### Conversion of D-galactose after the enzymatic hydrolysis of lactose in milk using micro-organisms expressing the arabinose isomerase trapped in alginate beads

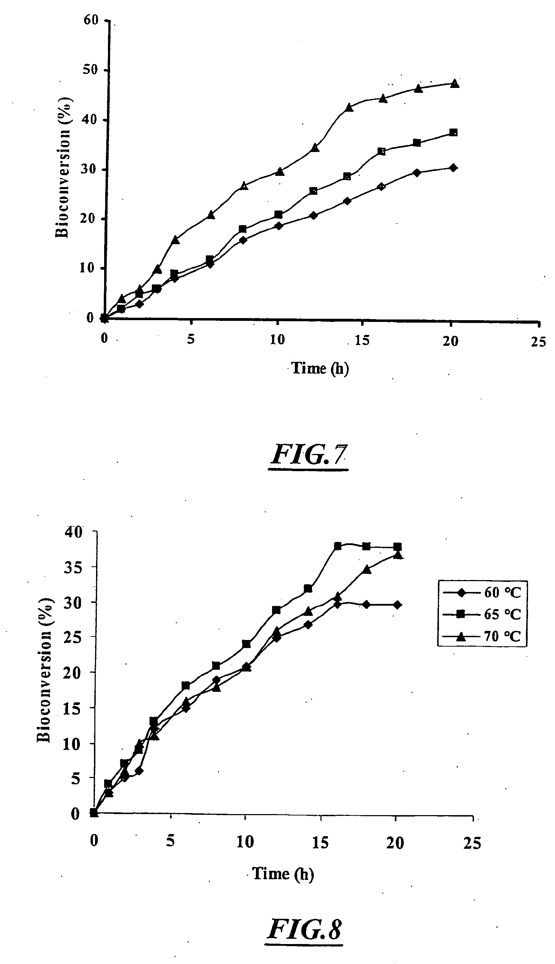

Milk was enriched in D-galactose by the enzymatic hydrolysis of lactose into D-glucose and D-galactose at one to one ratio. At 25 h, a concentration of D-glucose reached 25 g/L. At that time, the alginate beads containing the strains expressing the wild-type L-AI US100 or mutated L-AI N175H Q268K were added to the treated milk. The kinetics of bioconversion of D-Galactose in D-tagatose was then followed during the incubation at three temperatures: 60, 65 and 70°C figures 7, 8). For the wild-type enzyme, a conversion rate of 48% was obtained at the highest tested temperature after 20 h of incubation whereas as expected for the double mutated L-AI which is less thermophile than the wild-type enzyme exhibited a lower maximal conversion rate at the same time point (38%).

Altogether, these data established for the first time that cells expressing L-AI and embedded in a matrix can be used efficiently for the production of D-tagatose in milk enriched in D-galactose by an enzymatic treatment. Moreover, this type of setting could allow repeated use of the matrix. Consequently, the overall enzyme stability may become a crucial factor in this case.

## Claims

1. Use of a L-arabinose isomerase for converting D-galactose into D-tagatose directly in a dairy product which contains D-galactose, wherein said dairy product which contains D-galactose is selected fom the group consisting of milk, milk supplemented with D-galactose, milk undergoing lactic acid fermentation, a fermented milk, and milk in which lactose was enzymatically processed.

2. The use according to claim 1, wherein said L-arabinose isomerase is derived from a bacterium selected from the group consisting of a bacterium of the genus *Thermus, Thermotoga, Geobacillus, Lactobacillus* and *Bacillus,* of *Alicyclobacillus acidocaldarius,* and of a lactic acid bacterium.

3. The use according to any one of claims 1 to 2, wherein said L-arabinose isomerase is selected from the group consisting of AAAI, AAAI-K269E, BHAI, BHAI-E268K, L-AI US 100, L-AI US 100 comprising a substitution Q to K at position 268 of SEQ ID NO:2, L-AI US 100 comprising a substitution N to H at position 175 of SEQ ID NO:2, and L-AI US 100 comprising a substitution N to H at position 175 and Q to K at position 268 of SEQ ID NO:2.

4. The use according to any of claims 1 to 3, wherein at least one L-arabinose isomerase-expressing microorganism is added or contacted into the dairy product which contains D-galactose.

5. The use according to claim 4, wherein said L-arabinose isomerase-expressing microorganism was transformed to express L-arabinose isomerase or naturally expresses L-arabinose isomerase.

6. The use according to claim 4 or 5, wherein said L-arabinose isomerase-expressing microorganism is a lactic acid bacterium.

7. A method for converting D-galactose produced by lactic acid fermentation of milk into D-tagatose which comprises the steps of :
a) adding into the milk at least one L-arabinose isomerase and/or one L-arabinose isomerase-expressing microorganism acceptable for food consumption; and
b) operating lactic acid fermentation of milk;
whereby D-galactose produced in the milk by lactic acid fermentation is converted into D-tagatose.

8. A method for converting D-galactose produced by lactic acid fermentation of milk into D-tagatose, which method comprises the steps of:
a) contacting the milk with at least one L-arabinose isomerase and/or one L-arabinose isomerase-expressing microorganism; and
b) operating lactic acid fermentation of milk;
whereby D-galactose produced in the milk during lactic acid fermentation is converted into D-tagatose.

9. The method according to claim 7 or 8, wherein said L-arabinose isomerase-expressing microorganism is a lactic acid bacterium.

10. The method according to any one of claims 7 to 9, wherein said L-arabinose isomerase is selected from the group consisting of AAAI, AAAI-K269E, BHAI, BHAI-E268K, L-AI US 100, L-AI US 100 comprising a substitution Q to K at position 268 of SEQ ID NO:2, L-AI US 100 comprising a substitution N to H at position 175 of SEQ ID NO:2, and L-AI US 100 comprising a substitution N to H at position 175 and Q to K at position 268 of SEQ ID NO:2.

11. The method according to any of claims 7 to 10, wherein said L-arabinose isomerase-expressing microorganism was transformed to express L-arabinose isomerase or naturally expresses L-arabinose isomerase.

12. A method of producing a sweetened fermented dairy product which comprises the steps of:
a) adding at least one L-arabinose isomerase and/or one L-arabinose isomerase-expressing microorganism acceptable for food consumption into said fermented dairy product; or
b) contacting said dairy product containing D-galactose with at least one L-arabinose isomerase and/or one L-arabinose isomerase-expressing microorganism;
whereby a sweetened fermented dairy product is obtained.

13. A sweetening milk additive which comprises a L-arabinose isomerase and/or a L-arabinose isomerase-expressing microorganism.

14. A bacterium acceptable for food consumption which expresses an exogenous L-arabinose isomerase.

15. The bacterium according to claim 15, which has been transformed with a vector comprising a nucleic acid sequence encoding a L-arabinose isomerase.

16. The bacterium according to claim 14 or 15, which is a lactic acid bacterium.

17. The bacterium according to any one of claims 14 to 16, wherein said L-arabinose isomerase is selected from the group consisting of AAAI, AAAI-K269E, BHAI, BHAI-E268K, L-AI US 100, L-AI US 100 comprising a substitution Q to K at position 268 of SEQ ID NO:2, L-AI US 100 comprising a substitution N to H at position 175 of SEQ ID NO:2, and L-AI US 100 comprising a substitution Q to K at position 268 and N to H at position 175 of SEQ ID NO:2.

18. A vector suitable for transformation of a bacterium, which vector comprises a nucleic acid sequence encoding a L-arabinose isomerase selected from the L-arabinose isomerases L-AI US 100 comprising a substitution Q to K at position 268 of SEQ ID NO:2, L-AI US 100 comprising a substitution N to H at position 175 of SEQ ID NO:2, and L-AI US 100 comprising a substitution N to H at position 175 and Q to K at position 268 of SEQ ID NO:2.

19. A mutated L-arabinose isomerase US100 of SEQ ID NO:2 comprising a substitution Q to K at position 268 of SEQ ID NO:2 and/or a substitution N to H at position 175.

20. An isolated nucleic acid comprising a sequence encoding the mutated L-arabinose isomerase US100 of SEQ ID NO:2 comprising a substitution Q to K at position 268 of SEQ ID NO:2 and/or a substitution N to H at position 175.

## Patentansprüche

1. Einsatz einer L-Arabinose-Isomerase zur unmittelbaren Umwandlung von D-Galaktose in D-Tagatose in einem Molkereiprodukt, das D-Galaktose enthält, wobei das D-Galaktose enthaltende Molkereiprodukt aus der nachfolgenden Gruppe gewählt ist: Milch, mit D-Galaktose ergänzte Milch, Milch, die einer Milchsäurefermentation unterzogen wird, fermentierte Milch sowie Milch, in der Laktose enzymatisch verarbeitet wurde.

2. Einsatz nach Anspruch 1, wobei die L-Arabinose-Isomerase aus einem Bakterium aus der nachfolgenden Gruppe abgeleitet ist: Bakterium der Gattung Thermus, Thermotoga, Geobacillus, Lactobacillus und Bacillus, von Alicyclobacillus acidocaldarius und ein Milchsäurebakterium.

3. Einsatz nach einem der Ansprüche 1 - 2, wobei die L-Arabinose-Isomerase aus der nachfolgenden Gruppe gewählt ist: AAAI, AAAI-K269E, BHAI, BHAI-E268K, L-AI US 100, LAI US 100, umfassend eine Q-zu-K-Substitution an der Stelle 268 der SEQ ID NR. 2, L-AI US 100, umfassend eine N-zu-H-Substitution an der Stelle 175 der SEQ ID NR. 2 und L-AI US 100, umfassend eine N-zu-H-Substitution an der Stelle 175 und eine Q-zu-Ka-Substitution an der Stelle 268 der SEQ ID NR. 2.

4. Einsatz nach einem der Ansprüche 1 - 3, wobei mindestens ein L-Arabinose-Isomerase exprimierender Mikroorganismus dem D-Galaktose enthaltenden Molkereiprodukt hinzugegeben wird oder dieses kontaktiert.

5. Einsatz nach Anspruch 4, wobei der L-Arabinose-Isomerase exprimierende Mikroorganismus in L-Arabinose-Isomerase umgewandelt wurde oder von Natur aus L-Arabinose-Isomerase exprimiert.

6. Einsatz nach Anspruch 4 oder 5, wobei der L-Arabinose-Isomerase exprimierende Mikroorganismus ein Milchsäurebakterium ist.

7. Verfahren zur Umwandlung von durch die Milchsäurefermentation von Milch hergestellter D-Galaktose in D-Tagatose, umfassend die nachfolgenden Schritte:
a) Hinzugeben mindestens einer L-Arabinose-Isomerase und/oder eines als Nahrungsmittel annehmbaren L-Arabinose-Isomerase exprimierenden Mikroorganismus zur Milch; und
b) Durchführen einer Milchsäurefermentation der Milch;
wobei die in der Milch durch Milchsäurefermentation hergestellte D-Galaktose in D-Tagatose umgewandelt wird.

8. Verfahren zur Umwandlung von durch die Milchsäurefermentation von Milch hergestellter D-Galaktose in D-Tagatose, umfassend die nachfolgenden Schritte:
a) Kontaktieren der Milch mit mindestens einer L-Arabinose-Isomerase und/oder einem L-Arabinose-Isomerase exprimierenden Mikroorganismus; und
b) Durchführen einer Milchsäurefermentation der Milch;
wobei die in der Milch bei der Milchsäurefermentation hergestellte D-Galaktose in D-Tagatose umgewandelt wird.

9. Verfahren nach Anspruch 7 oder 8, wobei der L-Arabinose-Isomerase exprimierende Mikroorganismus ein Milchsäurebakterium ist.

10. Verfahren nach einem der Ansprüche 7 - 9, wobei die L-Arabinose-Isomerase aus der nachfolgenden Gruppe gewählt ist: AAAI, AAAI-K269E, BHAI, BHAI-E268K, L-AI US 100, LAI US 100, umfassend eine Q-zu-K-Substitution an der Stelle 268 der SEQ ID NR. 2, L-AI US 100, umfassend eine N-zu-H-Substitution an der Stelle 175 der SEQ ID NR. 2 und L-AI US 100, umfassend eine N-zu-H-Substitution an der Stelle 175 und eine Q-zu-K-Substitution an der Stelle 268 der SEQ ID NR. 2.

11. Verfahren nach einem der Ansprüche 7 - 10, wobei der L-Arabinose-Isomerase exprimierende Mikroorganismus in L-Arabinose-Isomerase umgewandelt wurde oder von Natur aus L-Arabinose-Isomerase exprimiert.

12. Verfahren zur Herstellung eines versüßten fermentierten Molkereiprodukts, umfassend die nachfolgenden Schritte:
a) Hinzugeben mindestens einer L-Arabinose-Isomerase und/oder eines als Nahrungsmittel annehmbaren L-Arabinose-Isomerase exprimierenden Mikroorganismus zum fermentierten Molkereiprodukt; und
a) Kontaktieren des D-Galaktose enthaltenden Molkereiprodukts mit mindestens einer L-Arabinose-Isomerase und/oder einem L-Arabinose-Isomerase exprimierenden Mikroorganismus;
wobei sich ein versüßtes fermentiertes Molkereiprodukt ergibt.

13. Versüßender Zusatzstoff für Milch, umfassend eine L-Arabinose-Isomerase und/oder einen L-Arabinose-Isomerase exprimierenden Mikroorganismus.

14. Als Nahrungsmittel annehmbares Bakterium, welches eine exogene L-Arabinose-Isomerase exprimiert.

15. Bakterium nach Anspruch 15, das mit einem Vektor umgewandelt wurde, der eine eine L-Arabinose-Isomerase codierende Nukleinsäuresequenz umfasst.

16. Bakterium nach Anspruch 14 oder 15, bei dem es sich um ein Milchsäurebakterium handelt.

17. Bakterium nach einem der Ansprüche 14 - 16, wobei die L-Arabinose-Isomerase aus der nachfolgenden Gruppe gewählt ist: AAAI, AAAI-K269E, BHAI, BHAI-E268K, L-AI US 100, LAI US 100, umfassend eine Q-zu-K-Substitution an der Stelle 268 der SEQ ID NR. 2, L-AI US 100, umfassend eine N-zu-H-Substitution an der Stelle 175 der SEQ ID NR. 2 und L-AI US 100, umfassend eine N-zu-H-Substitution an der Stelle 268 und eine Q-zu-K-Substitution an der Stelle 175 der SEQ ID NR. 2.

18. Zur Umwandlung eines Bakteriums geeigneter Vektor, welcher eine eine L-Arabinose-Isomerase codierende Nukleinsäuresequenz umfasst, die aus der nachfolgenden Gruppe gewählt ist: L-Arabinose-Isomerasen L-AI US 100, LAI US 100, umfassend eine Q-zu-K-Substitution an der Stelle 268 der SEQ ID NR. 2, L-AI US 100, umfassend eine N-zu-H-Substitution an der Stelle 175 der SEQ ID NR. 2 und L-AI US 100, umfassend eine N-zu-H-Substitution an der Stelle 175 und eine Q-zu-K-Substitution an der Stelle 268 der SEQ ID NR. 2.

19. Mutierte L-Arabinose-Isomerase US100 der SEQ ID NR. 2, umfassend eine Q-zu-K-Substitution an der Stelle 268 der SEQ ID NR. 2 und/oder eine N-zu-H-Substitution an der Stelle 175.

20. Isolierte Nukleinsäure, umfassend eine die mutierte L-Arabinose-Isomerase US100 der SEQ ID NR. 2, umfassend eine Q-zu-K-Substitution an der Stelle 268 der SEQ ID NR. 2 und/oder eine N-zu-H-Substitution an der Stelle 175, codierende Sequenz.

## Revendications

1. Utilisation d'une L-arabinose isomérase pour convertir le D-galactose en D-tagatose directement dans un produit laitier qui contient du D-galactose, dans laquelle ledit produit laitier qui contient du D-galactose est choisi dans le groupe constitué par le lait, le lait complémenté de D-galactose, le lait subissant une fermentation lactique, un lait fermenté, et le lait dans lequel le lactose a subi une transformation enzymatique.

2. Utilisation selon la revendication 1, dans laquelle ladite L-arabinose isomérase dérive d'une bactérie choisie dans le groupe constitué par une bactérie du genre *Thermus, Thermotoga, Geobacillus, Lactobacillus* et *Bacillus, Alicyclobacillus Acidocaldarius,* et un ferment lactique.

3. Utilisation selon l'une quelconque des revendications 1 et 2, dans laquelle ladite L-arabinose isomérase est choisie dans le groupe constitué par AAAI, AAAI-K269E, BHAI, BHAI-E268K, L-AI US 100, L-AI US 100 comprenant une substitution de Q en K à la position 268 de la SEQ ID NO: 2, L-AI US 100 comprenant une substitution de N en H à la position 175 de la SEQ ID NO : 2, et L-AI US 100 comprenant des substitutions de N en H à la position 175 et de Q en K à la position 268 de la SEQ ID NO : 2.

4. Utilisation selon l'une quelconque des revendications 1 à 3, dans laquelle au moins un microorganisme exprimant une L-arabinose isomérase est ajouté ou mis en contact dans le produit laitier qui contient du D-galactose.

5. Utilisation selon la revendication 4, dans laquelle ledit microorganisme exprimant une L-arabinose isomérase a été transformé pour exprimer une L-arabinose isomérase, ou exprime naturellement une L-arabinose isomérase.

6. Utilisation selon la revendication 4 ou 5, dans laquelle ledit microorganisme exprimant une L-arabinose isomérase est un ferment lactique.

7. Procédé pour convertir du D-galactose produit par fermentation lactique du lait en D-tagatose, qui comprend les étapes consistant à :
a) ajouter dans le lait au moins une L-arabinose isomérase et/ou un microorganisme exprimant une L-arabinose isomérase acceptable pour un usage alimentaire ; et
b) effectuer une fermentation lactique du lait ;
grâce à quoi le D-galactose produit dans le lait par fermentation lactique est converti en D-tagatose.

8. Procédé pour convertir du D-galactose produit par fermentation lactique du lait en D-tagatose, qui comprend les étapes consistant à :
a) mettre le lait en contact avec au moins une L-arabinose isomérase et/ou un microorganisme exprimant une L-arabinose isomérase ; et
b) effectuer une fermentation lactique du lait ;
grâce à quoi le D-galactose produit dans le lait par fermentation lactique est converti en D-tagatose.

9. Procédé selon la revendication 7 ou 8, dans lequel ledit microorganisme exprimant une L-arabinose isomérase est un ferment lactique.

10. Procédé selon l'une quelconque des revendications 7 à 9, dans lequel ladite L-arabinose isomérase est choisie dans le groupe constitué par AAAI, AAAI-K269E, BHAI, BHAI-E268K, L-AI US 100, L-AI US 100 comprenant une substitution de Q en K à la position 268 de la SEQ ID NO : 2, L-AI US 100 comprenant une substitution de N en H à la position 175 de la SEQ ID NO : 2, et L-AI US 100 comprenant des substitutions de N en H à la position 175 et de Q en K à la position 268 de la SEQ ID NO : 2.

11. Procédé selon l'une quelconque des revendications 7 à 10, dans lequel ledit microorganisme exprimant une L-arabinose isomérase a été transformé pour exprimer une L-arabinose isomérase, ou exprime naturellement une L-arabinose isomérase.

12. Procédé pour produire un produit laitier fermenté sucré, qui comprend les étapes consistant à :
a) ajouter au moins une L-arabinose isomérase et/ou un microorganisme exprimant une L-arabinose isomérase acceptable pour un usage alimentaire dans ledit produit laitier fermenté ; ou
b) mettre ledit produit laitier contenant du D-galactose en contact avec au moins une L-arabinose isomérase et/ou un microorganisme exprimant une L-arabinose isomérase ;
grâce à quoi un produit laitier fermenté sucré est obtenu.

13. Additif sucrant pour le lait qui comprend une L-arabinose isomérase et/ou un microorganisme exprimant une L-arabinose isomérase.

14. Bactérie acceptable pour un usage alimentaire qui exprime une L-arabinose isomérase exogène.

15. Bactérie selon la revendication 14, qui a été transformée avec un vecteur comprenant une séquence d'acide nucléique codant une L-arabinose isomérase.

16. Bactérie selon la revendication 14 ou 15, qui est un ferment lactique.

17. Bactérie selon l'une quelconque des revendications 14 à 16, dans laquelle ladite L-arabinose isomérase est choisie dans le groupe constitué par AAAI, AAAI-K269E, BHAI, BHAI-E268K, L-AI US 100, L-AI US 100 comprenant une substitution de Q en K à la position 268 de la SEQ ID NO: 2, L-AI US 100 comprenant une substitution de N en H à la position 175 de la SEQ ID NO : 2, et L-AI US 100 comprenant des substitutions de N en H à la position 175 et de Q en K à la position 268 de la SEQ ID NO : 2.

18. Vecteur convenant pour la transformation d'une bactérie, lequel vecteur comprend une séquence d'acide nucléique codant une L-arabinose isomérase choisie parmi les L-arabinose isomérases L-AI US 100 comprenant une substitution de Q en K à la position 268 de la SEQ ID NO : 2, L-AI US 100 comprenant une substitution de N en H à la position 175 de la SEQ ID NO : 2, et L-AI US 100 comprenant des substitutions de N en H à la position 175 et de Q en K à la position 268 de la SEQ ID NO : 2.

19. L-arabinose isomérase US100 de la SEQ ID NO : 2 mutée comprenant une substitution de Q en K à la position 268 de la SEQ ID NO: 2 et/ou une substitution de N en H à la position 175.

20. Acide nucléique isolé comprenant une séquence codant la L-arabinose isomérase US100 de la SEQ ID NO : 2 mutée comprenant une substitution de Q en K à la position 268 de la SEQ ID NO : 2 et/ou une substitution de N en H à la position 175.
